# EUROPÄISCHE PATENTSCHRIFT

(11) **EP 0 986 526 B1**
(45) Veröffentlichungstag und Bekanntmachung des Hinweises auf die Patenterteilung: **30.01.2002**
(21) Anmeldenummer: 98929426.9
(22) Anmeldetag: 05.06.1998
(51) Int. Cl.: C07B 41/00, C07D 301/12, C07D 301/19

(54) **VERFAHREN ZUR OXIDATION EINER MINDESTENS EINE C-C-DOPPELBINDUNG AUFWEISENDEN ORGANISCHEN VERBINDUNG**
METHOD FOR OXIDIZING AN ORGANIC COMPOUND CONTAINING AT LEAST ON C-C DOUBLE BOND
PROCEDE POUR L'OXYDATION D'UN COMPOSE ORGANIQUE POSSEDANT AU MOINS UNE LIAISON DOUBLE C-C

(30) Priorität: 06.06.1997 DE 19723950
(43) Veröffentlichungstag der Anmeldung: 22.03.2000
(73) Patentinhaber: BASF AKTIENGESELLSCHAFT, 67056 Ludwigshafen (DE)
(72) Erfinder: GROSCH, Georg, Heinrich, D-67098 Bad Dürkheim (DE); MÜLLER, Ulrich, D-67435 Neustadt (DE); WALCH, Andreas, D-69120 Heidelberg (DE); RIEBER, Norbert, D-68259 Mannheim (DE); FISCHER, Martin, D-67071 Ludwigshafen (DE); QUAISER, Stefan, D-67117 Limburgerhof (DE); HARDER, Wolfgang, D-69469 Weinheim (DE); ELLER, Karsten, D-67061 Ludwigshafen (DE); BASSLER, Peter, D-68519 Viernheim (DE); WENZEL, Anne, D-76676 Graben-Neudorf (DE); KAIBEL, Gerd, D-68623 Lampertheim (DE); STAMMER, Achim, D-67251 Freinsheim (DE); HENKELMANN, Jochem, D-68165 Mannheim (DE); BÖTTCHER, Arnd, D-67227 Frankenthal (DE); TELES, Joaquim, Henrique, D-67122 Altrip (DE); SCHULZ, Michael, D-67067 Ludwigshafen (DE); TREIBER, Gert, D-67549 Worms (DE)
(74) Vertreter: Isenbruck, Günter, Dr.
(86) Internationale Anmeldenummer: EP9803395
(87) Internationale Veröffentlichungsnummer: WO9855430

(56) Entgegenhaltungen:
- EP-A- 0 743 094
- DE-C- 19 528 220
- US-A- 5 374 747
- US-A- 5 384 418
- US-A- 5 463 090
- US-A- 5 599 955

## Beschreibung

Die vorliegende Erfindung betrifft ein Verfahren zur Oxidation einer mindestens eine C-C-Doppelbindung aufweisenden organischen Verbindung oder eines Gemischs aus zwei oder mehr davon, wobei die mindestens eine C-C-Doppelbindung aufweisende organische Verbindung oder das Gemisch aus zwei oder mehr davon mit einem Hydroperoxid in Gegenwart eines Zeolith-Katalysators umgesetzt wird, dieser Katalysator regeneriert wird, und nach der Regenerierung des Katalysators dieser wieder zur oben genannten Umsetzung verwendet wird.

Verfahren zur Oxidation einer mindestens eine C-C-Doppelbindung aufweisenden organischen Verbindung, insbesondere von Olefinen und unter diesen bevorzugt Propylen mittels eines Hydroperoxids sind bekannt.

So beschreibt die US-A-5,374,747 ein derartiges Epoxidationsverfahren unter Verwendung eines titanhaltigen Molekularsiebs, das eine Struktur aufweist, die mit Zeolith-β isomorph ist, sowie die Herstellung eines derartigen Molekularsiebs an sich.

Die US-A 5,384,418 beschreibt ein integriertes Verfahren zur Herstellung von Epoxiden durch Umsetzung eines Hydroperoxids mit einer ethylenisch ungesättigten Verbindung in Gegenwart eines Ti-Silikalits.

Weitere Verfahren zur Herstellung von Epoxiden in Gegenwart von Zeolith-Katalysatoren sind u.a. in der US-A 5,463,090 und der EP-A 0 230 949 beschrieben, wobei in ersterer das zur Oxidation verwendete Wasserstoffperoxid aus einem Anthrachinonprozeß gewonnen wird, während in letztgenannter Druckschrift die Epoxidation von Propylen mit Wasserstoffperoxid in Gegenwart von darin definierten Titansilikaliten beschrieben wird.

Gemäß der US-A 5,599,955 kann das für derartige Oxidationen meist eingesetzte Propylen ausgehend von Synthesegas gewonnen werden. Die US-A 5,599,956 beschreibt ein Verfahren zur Herstellung von Propylenoxid, bei dem das Propylen durch Steamcracken, katalytisches Cracken oder katalytische Dehydrierung gewonnen wird.

Bekanntermaßen bilden sich bei diesen katalytischen Umsetzungen, insbesondere bei der Verwendung von Katalysatoren, die Mikroporen aufweisen, wie z.B. bei Zeolith-Katalysatoren wie Titansilikalit oder titanhaltigem Zeolith-β, nach einiger Zeit organische Beläge, die zu einer teilweisen oder vollständigen Deaktivierung der Katalysatoren führen.

Diese organischen Beläge können durch Calcinieren des Katalysators oder durch Waschen mit Lösungsmittel zum größten Teil entfernt werden (M.G. Clerici, G. Bellussi, U. Romano, J. Catal., 129 (1991), S. 159 - 167; JP-A 03 114 536).

Die EP-A 0 743 094 beschreibt ein Verfahren zur Regenerierung eines Ti enthaltenden Molekularsiebs, das das Erwärmen des Molekularsiebs bei einer Temperatur von mehr als 150 °C und weniger als 400 °C umfaßt. Dort wird auch die mögliche Verwendung des so regenerierten Katalysators für die Umsetzung organischer Verbindungen, beispielsweise für die Hydroxylierung von aromatischen Verbindungen, Ammoxidation von Ketonen, Oxidation von gesättigten Kohlenwasserstoffen zu Alkoholen und Ketonen und auch zur Olefin-Epoxidation beschrieben. Die DE-A 44 25 672 beschreibt einen Oxidationskatalysator auf Basis von Titan- oder Vanadiumsilikaten mit Zeolith-Struktur, sowie ein Verfahren zur Herstellung von Epoxiden aus Olefinen, Wasserstoff und Sauerstoff unter Verwendung des darin beschriebenen Katalysators. Dort wird auch angegeben, daß der darin beschriebene Katalysator regeneriert werden kann.

Auch die weiter oben bereits diskutierte US-A 5,599,955 spricht die Möglichkeit einer Regenerierung des im Rahmen des dort beschriebenen Verfahrens verwendeten Katalysators an, ohne jedoch Details bezüglich der Durchführung der Regenerierung zu geben.

Gemäß der DE-A-195 28220 werden zur Herstellung von Epoxiden aus Olefinen und Wasserstoffperoxid als Katalysator verwendete, Titanatome enthaltande Zeolithe regeneriert, indem man den gebrauchten Katalysator in Abwesenheit von Olefinen mit Wasserstoffperoxid behandelt und den regenerierten Katalysator erneut zur Epoxidierung einsetzt.

Wie sich aus obigem ergibt, beschreibt der Stand der Technik zwar eingehend integrierte Verfahren zur Herstellung von Epoxiden, die Frage nach einer praktikablen Regenerierung des deaktivierten Katalysators und die sinnvolle Einbindung einer derartigen Stufe in den Gesamtprozeß bleibt jedoch ungelöst. Dabei ist gerade diese Stufe und die Einbindung derselben in das Gesamtverfahren für die Wirtschaftlichkeit eines derartigen Verfahrens von entscheidender Bedeutung. Regenerierungen, wie sie in der EP-A 0 743 094 beschrieben sind, können zwar prinzipiell durchgeführt werden, sind jedoch aufgrund der dort verwendeten niedrigen Temperaturen und der dadurch erzwungenen langen Regenerierungsdauer wirtschaftlich nicht durchführbar.

Somit liegt der vorliegenden Erfindung die Aufgabe zugrunde, ein Verfahren zur Oxidation einer mindestens eine C-C-Doppelbindung aufweisenden organischen Verbindung bereitzustellen, das die Regenerierung des im Rahmen dieses Verfahrens verwendeten Katalysators und die anschließende Verwendung des regenerierten Katalysators zur weiteren Umsetzung innerhalb des Verfahrens ermöglicht.

Diese Aufgabe wird durch das erfindungsgemäße Verfahren gelöst.

Somit betrifft die vorliegende Erfindung ein Verfahren zur Oxidation einer mindestens eine C-C-Doppelbindung aufweisenden organischen Verbindung oder eines Gemischs aus zwei oder mehr davon, das die folgenden Stufen umfaßt:
(I) Herstellung eines Hydroperoxids,
(II) Umsetzung einer mindestens eine C-C-Doppelbindung aufweisenden organischen Verbindung oder eines Gemischs aus zwei oder mehr davon mit dem in Stufe (I) hergestellten Hydroperoxid in Gegenwart eines Zeolith-Katalysators,
(III) Regenerierung des in Stufe (II) verwendeten, zumindest teilweise deaktivierten Zeolith-Katalysators, und
(IV) Durchführung der Umsetzung gemäß Stufe (II) unter Verwendung eines Zeolith-Katalysators, der den in Stufe (III) regenerierten Katalysator enthält.

### Stufe (I)

Diese Stufe betrifft die Herstellung eines Hydroperoxids. Der Begriff "Hydroperoxid" im Kontext der vorliegenden Anmeldung bezeichnet sowohl Wasserstoffperoxid als auch organische Verbindungen der allgemeinen Formel R-O-OH, wobei R einen Alkylrest, einen Cycloalkylrest, einen Aralkylrest oder einen aromatischen Rest darstellt.

Vorzugsweise wird im Rahmen des erfindungsgemäßen Verfahrens Wasserstoffperoxid verwendet.

Verfahren zur Herstellung der Hydroperoxide sind an sich bekannt und sollen im folgenden lediglich für die Synthese von Wasserstoffperoxid nochmals kurz erläutert werden.

Dabei erfolgt die Synthese des Wasserstoffperoxids vorzugsweise gemäß einem Anthrachinon-Verfahren oder direkt aus Wasserstoff und Sauerstoff an edelmetallhaltigen Katalysatoren.

Beim Anthrachinon-Verfahren wird eine im folgenden Arbeitslösung genannte Mischung hergestellt. Diese besteht aus einer Lösung eines 2-Alkylanthrachinons, bevorzugt 2-Ethyl-, 2-Butyl-, 2-Hexyl-, 2-Hexenyl-, besonders bevorzugt 2-Ethylanthrachinon in einem Lösungsmittelgemisch enthaltend ein Lösungsmittel für Chinon und ein Lösungsmittel für Hydrochinon. Das Lösungsmittel für Chinon wird im allgemeinen ausgewählt aus der Gruppe der aromatischen und alkylaromatischen Lösungsmittel, vorzugsweise Benzol, Toluol, Xylole oder höhere Alkylaromaten mit 6 bis 20, vorzugsweise 9 bis 11 Kohlenstoffatomen oder Gemischen aus zwei oder mehr davon, wobei derartige Gemische bevorzugt sind.

Das Lösungsmittel für Hydrochinon wird im allgemeinen ausgewählt aus der Gruppe der Alkylphosphate, Alkylphosphonate, Nonylalkohole, Alkylcyclohexanol-Ester, N,N-Dialkylcarbonylamide, Tetraalkylurethane oder N-Alkyl-2-pyrrolidon und Gemischen aus zwei oder mehr davon, wobei Tetrabutylharnstoff bevorzugt ist.

Die Arbeitslösung wird an einem handelsüblichen, mindestens ein Übergangsmetall enthaltenden Katalysator, vorzugsweise 0,5 bis 20 Gew.-% Pd auf Aktivkohle, weiter bevorzugt 2 bis 15 Gew.-% Pd auf Aktivkohle, mit Wasserstoff bei ungefähr 20 bis 100 °C, vorzugsweise ungefähr 40 bis 70 °C hydriert. Der Katalysator kann in Form einer Suspension oder eines Festbetts angeordnet sein.

Die daraus resultierende Hydrochinon-haltige Lösung wird in einer geeigneten Vorrichtung, beispielsweise einer Blasensäule, mit Sauerstoff, vorzugsweise mit Luft, weiter bevorzugt mit einem Sauerstoff- und Stickstoff enthaltenden Gemisch, bei dem der Sauerstoff im Unterschuß, bezogen auf das Gesamtgemisch, vorliegt, oxidiert. Die Oxidation erfolgt bei einer Reaktionstemperatur von ungefähr 20 bis ungefähr 100 °C, vorzugsweise bei ungefähr 35 bis ungefähr 60 °C, wobei die Oxidation solange durchgeführt wird, bis die Lösung einen konstanten Gehalt an Wasserstoffperoxid aufweist und das Hydrochinon komplett in Chinon umgewandelt wurde.

Das so erhaltene, Wasserstoffperoxid enthaltende Gemisch wird anschließend mit einem mit dem Lösungsmittelgemisch nicht mischbaren Lösungsmittel, vorzugsweise Wasser, Methanol, einem einwertigen Alkohol mit 2 bis 6 C-Atomen oder einem Gemisch aus zwei oder mehr davon, weiter bevorzugt mit Wasser extrahiert. Das so erhaltene Wasserstoffperoxid enthaltende Gemisch kann anschließend direkt in die Umsetzung gemäß Stufe (II) des erfindungsgemäßen Verfahrens eingesetzt werden. Eine derartige Aufarbeitung ist u.a. in der EP-B 0 549 013 beschrieben, die die Verwendung eines Gemischs aus Wasser und einem Alkohol, vorzugsweise Methanol, vorschlägt.

Ferner kann das im Rahmen der vorliegenden Erfindung zur Oxidation vorzugsweise verwendete Wasserstoffperoxid auch direkt aus den Elementen hergestellt werden. Wie sich aus der DE-A 196 42 770 und dem darin zitierten Stand der Technik ergibt, sind Verfahren zur Herstellung von Wasserstoffperoxid aus den Elementen Sauerstoff und Wasserstoff wohl bekannt. Vorzugsweise wird im Rahmen des erfindungsgemäßen Verfahrens die Synthese von Wasserstoffperoxid aus den Elementen gemäß dem in der DE-A 196 42 770 beschriebenen Verfahren durchgeführt, wobei diese Anmeldung vollumfänglich in die vorliegende Anmeldung durch Bezugnahme einbezogen wird.

Im folgenden sollen nun noch einmal kurz die wesentlichen Aspekte des dort beschriebenen Verfahrens erläutert werden.

Gemäß des dort beschriebenen Verfahrens wird Wasserstoffperoxid kontinuierlich durch die Umsetzung von Wasserstoff und Sauerstoff in Wasser und/oder C₁-C₃-Alkanolen als Reaktionsmedium an einem Katalysatorformkörper, der als aktive Komponente Palladium enthält, hergestellt. Gemäß dieses Verfahrens wird eine Wasserstoffperoxid-Lösung erhalten, die einen Gehalt an Wasserstoffperoxid von mindestens 2,5 Gew.-%, bezogen auf die Gesamtlösung, aufweist.

Unter Katalysatorformkörpern sind dabei Katalysatoren zu verstehen, bei denen die katalytisch aktive Komponente sich auf der Oberfläche speziell geformter Träger befindet. Derartige Träger können übliche Füllkörper, wie z.B. Raschig-Ringe, Sattelkörper, Pall® -Ringe, Drahtspiralen oder Maschendrahtringe sein, die aus unterschiedlichen Materialien, die sich für eine Beschichtung mit der aktiven Komponente eignen, aufgebaut sind. Details bezüglich der oben genannten Träger sind Römpp-Chemie-Lexikon, 9. Aufl., S. 1453f. zu entnehmen. Die mit der katalytisch aktiven Komponente versehenen Füllkörper werden als lose Schüttung in den Reaktor gegeben.

Bevorzugte Formkörper weisen Kanäle mit hydraulischen Radien (Definition s. VDI-Wärmeatlas, Abschnitt LE1) im Bereich von 1 bis 10 mm auf.

Bevorzugt werden Katalysatorformkörper, die in Form geordneter Packungen in den Reaktor eingebaut werden und die aufgrund einer Vielzahl von Durchströmungskanälen eine große Oberfläche, bezogen auf ihr Volumen, aufweisen, eingesetzt. Derartige Formkörper bezeichnet man als Katalysatormonolithe. Geeignete Reaktoren für die Darstellung des Wasserstoffperoxids gemäß dieses Verfahrens sind beispielsweise in der EP-A 0 068 862, EP-A 0 201 614 und der EP-A 0 448 884 beschrieben.

Ein weiteres Verfahren zur Herstellung von Wasserstoffperoxid, das ebenfalls in das erfindungsgemäße Verfahren als Stufe (I) einbezogen werden kann, wird in der WO 96/05138 beschrieben. Diese Anmeldung wird bzgl. des dort beschriebenen Verfahrens zur Herstellung von Wasserstoffperoxid und der dazu verwendeten Vorrichtung vollumfänglich durch Bezugnahme in die vorliegende Anmeldung einbezogen.

Gemäß des dort beschriebenen Verfahrens werden kleine Blasen von Wasserstoff und Sauerstoff in einen Flüssigkeitsstrom aus Wasser und einer anorganischen Säure in Gegenwart eines ein Metall der VIII. Nebengruppe des Periodensystems enthaltenden Katalysators eingebracht. Der Flüssigkeitsstrom weist dabei eine Geschwindigkeit von mindestens ungefähr 3 m/s (10 Fuß/s) auf, wodurch ein kontinuierlicher Bereich an fein verteilten Gasbläschen in einer kontinuierlichen Flüssigkeitsphase geschaffen wird. Bezüglich weiterer Details zu dieser Methode zur Herstellung von Wasserstoffperoxid wird auf die oben genannte Druckschrift verwiesen.

Ferner kann das im Rahmen des erfindungsgemäßen Verfahrens verwendete Wasserstoffperoxid durch Inkontaktbringen eines sekundären Alkohols, wie z.B. α-Methylbenzylalkohol, Isopropanol, 2-Butanol oder Cyclohexanol mit molekularem Sauerstoff unter Bedingungen, die geeignet sind, ein Gemisch zu erhalten, das einen sekundären Alkohol und Wasserstoffperoxid und/oder einen Wasserstoffperoxid-Vorläufer umfaßt, hergestellt werden. Typischerweise enthält ein derartiges Gemisch ein dem jeweils verwendeten sekundären Alkohol entsprechendes Keton, d.h. ein Keton, das das gleiche Kohlenstoffgerüst wie der verwendete sekundäre Alkohol aufweist, wie z.B. Acetophenon, Aceton oder Cyclohexanon, geringe Menge an Wasser und wechselnde Mengen anderer aktiver Sauerstoff-Verbindungen, wie z.B. organische Hydroperoxide.

Ferner kann das verwendete Wasserstoffperoxid unmittelbar vor oder während der Epoxidation in situ hergestellt werden, wie dies beispielsweise in der EP-B 0 526 945, JP-A 4 352 771, EP-B 0 469 662 und Ferrini et al. in "Catalytic Oxidation of Alkanes using Titanium Silicate in the Presence of in-situ Generated Hydrogen Ferroxide", DGMK Conference on Selective Oxidations in Petrochemistry, Sep. 16 - 18, 1992, S. 205 - 213, beschrieben wird.

### Stufe (II)

Diese Stufe des erfindungsgemäßen Verfahrens betrifft die Umsetzung einer mindestens eine C-C-Doppelbindung aufweisenden Verbindung oder eines Gemischs aus zwei oder mehr davon, mit dem in Stufe (I) hergestellten Hydroperoxid in Gegenwart eines Zeolith-Katalysators.

Der im Rahmen der vorliegenden Erfindung verwendete Begriff "C-C-Doppelbindung aufweisende organische Verbindung" umfaßt alle organischen Verbindungen, die mindestens eine C-C-Doppelbindung aufweisen. Es kann sich dabei um eine niedermolekulare organische Verbindung, d.h. eine Verbindung, die ein Molekulargewicht von bis zu ungefähr 500 aufweist, sowie ein Polymer, d.h. eine Verbindung, die ein Molekulargewicht von mehr als 500 aufweist, handeln. Vorzugsweise wird das erfindungsgemäße Verfahren jedoch für niedermolekulare organische Verbindungen der obenbeschriebenen Art eingesetzt. Dabei kann es sich um lineare, verzweigtkettige oder cyclische Verbindungen handeln, die aromatische, aliphatische, cycloaliphatische Gruppen, sowie eine Kombination aus zwei oder mehr davon, aufweisen können. Vorzugsweise weist die eingesetzte organische Verbindung 2 bis 30 Kohlenstoffatome, weiter bevorzugt 2 bis 10 Kohlenstoffatome, auf. Sie ist weiter bevorzugt ein aliphatisches Monoolefin. Es ist jedoch auch möglich, daß die eingesetzte organische Verbindung mehr als eine ethylenisch ungesättigte Doppelbindung aufweist, wie dies beispielsweise in Dienen oder Trienen der Fall ist. Sie kann zusätzliche funktionelle Gruppen, wie z.B. ein Halogenatom, eine Carboxylgruppe, eine Estergruppe, eine Hydroxylgruppe, eine Etherbrücke, eine Sulfidbrücke, eine Carbonylgruppe, eine Cyanogruppe, eine Nitrogruppe, eine Aminogruppe oder eine Kombination aus zwei oder mehr davon enthalten. Die Doppelbindung kann endständig oder innenliegend sein. Ferner kann sie Bestandteil einer cyclischen Struktur sein, wie dies beispielsweise bei Cyclohexen der Fall ist. Es kann auch ein Gemisch aus zwei oder mehr derartiger Verbindungen eingesetzt werden.

Weitere Beispiele für geeignete organische Verbindungen schließen ungesättigte Fettsäuren oder deren Derivate, wie z.B. Ester und Glyceride derartiger ungesättigter Fettsäuren, sowie Oligomere oder Polymere ungesättigter organischer Verbindungen, wie z.B. Polybutadien, ein.

Beispiele derartiger organischer Verbindungen schließen die folgenden ein: Ethylen, Propylen, 1-Buten, cis- und trans-2-Buten, iso-Butylen, Butadien, Pentene, Isopren, 1-Hexen, 3-Hexen, 1-Hepten, 1-Octen, Diisobutylen, 1-Nonen, 1-Decen, Camphen, 1-Undecen, 1-Dodecen, 1-Tridecen, 1-Tetradecen, 1-Pentadecen, 1-Hexadecen, 1-Heptadecen, 1-Octadecen, 1-Nonadecen, 1-Eicosen, Di-, Tri- oder Tetramere des Propylens, Styrol sowie andere vinylaromatische organische Verbindungen mit mindestens einer C-C-Doppelbindung, Diphenylethylen, Polybutadien, Polyisopren, Cyclopenten, Cyclohexen, Cyclohepten, Cycloocten, Cyclooctadien, Cycoldodecen, Cyclododecatrien, Dicyclopentadien, Methylencyclopropan, Methylencyclopentan, Methylencyclohexan, Vinylcyclohexan, Vinylcyclohexen, Methallylketon, Allylchlorid, Allylbromid, Acrylsäure, Methacrylsäure, Crotonsäure, Vinylessigsäure, Crotylchlorid, Methallylchlorid, Dichlorbutene, Allylalkohol, Allylcarbonat, Allylacetat, Alkylacrylate und -methacrylate, Diallylmaleat, Diallylphthalat, ungesättigte Triglyceride, wie z.B. Sojaöl, ungesättigte Fettsäuren, wie z.B. Ölsäure, Linolsäure, Linolensäure, Ricinolsäure sowie deren Ester einschließlich der Mono-, Di- und Triglyceridester.

Ferner können auch Gemische aus zwei oder mehr derartigen Verbindungen, insbesondere Gemische der oben exemplarisch aufgeführten Verbindungen, eingesetzt werden.

Demnach betrifft die vorliegende Erfindung insbesondere ein Verfahren der hier in Rede stehenden Art, wobei die mindestens eine C-C-Doppelbindung aufweisende organische Verbindung ausgewählt wird aus der Gruppe bestehend aus einem linearen oder verzweigtkettigen aliphatischen, einem linearen oder verzweigtkettigen aromatischen, einem linearen oder verzweigtkettigen cycloaliphatischen Olefin, mit jeweils bis zu 30 C-Atomen und einem Gemisch aus zwei oder mehr davon.

Insbesondere eignet sich das erfindungsgemäße Verfahren zur Umsetzung niedermolekularer Olefine, wie z. B. Ethylen, Propylen, sowie der Butene, wobei insbesondere Propylen umgesetzt wird.

Als Katalysatoren werden in Stufe (II) des erfindungsgemäßen Verfahrens Übergangsmetall-haltige, mikroporöse und/oder mesoporöse und/oder makroporöse Festkörper eingesetzt.

Insbesondere bei der Oxidation niedermolekularer Verbindungen werden bevorzugt Übergangsmetall-haltige mikroporöse Festkörper benutzt, besonders bevorzugt werden Zeolithe, die Übergangsmetalle enthalten, weiter bevorzugt ein Titan-, Zirkonium-, Chrom-, Niob-, Eisen- oder Vanadium-haltiger Zeolith und insbesondere ein Titansilicalit eingesetzt.

Zeolithe sind bekanntermaßen kristalline Allumosilicate mit geordneten Kanalund Käfigstrukturen, die Mikroporen aufweisen. Der Begriff "Mikroporen", wie er im Rahmen der vorliegenden Erfindung verwendet wird, entspricht der Definition in "Pure Appl. Chem." 45, S. 71 ff., insbesondere S. 79 (1976), und bezeichnet Poren mit einem Porendurchmesser von kleiner 2 nm. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier und D.H. Olson in "Atlas of Zeolithe Structure Types", Elsevier, 4. Auflage, London 1996.

Ferner existieren Zeolithe, die kein Aluminium enthalten und bei denen im Silicatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben, beispielsweise in der EP-A 0 311 983 oder der EP-A 0 405 978. Außer Silizium und Titan können solche Materialien auch zusätzliche Elemente wie Aluminium, Zirkonium, Zinn, Eisen, Kobalt, Nickel, Gallium, Bor oder geringe Mengen an Fluor enthalten.

In den beschriebenen Zeolithen kann das Titan desselben teilweise oder vollständig durch Vanadium, Zirkonium, Chrom, Niob oder Eisen ersetzt sein. Das molare Verhältnis von Titan und/oder Vanadium, Zirkonium, Chrom, Niob oder Eisen zur Summe aus Silizium und Titan und/oder Vanadium, Zirkonium, Chrom, Niob oder Eisen liegt in der Regel im Bereich von 0,01:1 bis 0,1:1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Üblicherweise stellt man die genannten Titan-, Zirkonium-, Chrom-, Niob-, Eisen- und Vanadiumzeolithe dadurch her, daß man eine wäßrige Mischung aus einer SiO₂-Quelle, einer Titan-, Zirkonium-, Chrom-, Niob-, Eisen bzw. Vanadium-Quelle, wie z.B. Titandioxid bzw. einem entsprechenden Vanadiumoxid, Zirkoniumalkoholat, Chromoxid, Nioboxid oder Eisenoxid und einer stickstoffhaltigen organischen Base als Templat ("Schablonen-Verbindung"), wie z.B. Tetrapropylammoniumhydroxid, gegebenenfalls noch unter Hinzufügen von basischen Verbindungen, in einem Druckbehälter unter erhöhter Temperatur im Zeitraum mehrerer Stunden oder einiger Tage umsetzt, wobei ein kristallines Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan, bzw. das Zirkonium, Chrom, Niob, Eisen und/oder Vanadium zumindest teilweise innerhalb des Zeolithgerüsts in wechselndem Anteil mit 4-, 5- oder 6-facher Koordination vor. Zur Verbesserung der katalytischen Verhaltens kann sich noch eine mehrmalige Waschbehandlung mit schwefelsaurer Wasserstoffperoxid-lösung anschließen, worauf das Titan- bzw. Zirkonium-, Chrom-, Niob-, Eisen-, Vanadiumzeolith-Pulver erneut getrocknet und gebrannt werden muß; daran kann sich eine Behandlung mit Alkalimetallverbindungen anschließen, um den Zeolith von der H-Form in die Kation-Form zu überführen. Das so hergestellte Titan- bzw. Zirkonium-, Chrom-, Niob-, Eisen-, Vanadiumzeolith-Pulver wird dann, wie nachstehend beschrieben, zu einem Formkörper verarbeitet.

Bevorzugte Zeolithe sind Titan-, Zirkonium-, Chrom-, Niob- oder Vanadium-zeolithe, weiter bevorzugt solche mit Pentasil-Zeolith-Struktur, insbesondere die Typen mit röntgenographischer Zuordnung zur BEA-, MOR-, TON-, MTW-, FER-, MFI-, MEL-, CHA-, ERI-, RHD-, GIS-, BOG-, NON-, EMT-, HEU-, KFI-, FAU-, DDR-, MTT-, RUT-, LTL-, MAZ-, GME-, NES-, OFF-, SGT-, EUO-, MFS-, MCM-22- oder MFI/MEL-Mischstruktur. Zeolithe dieses Typs sind beispielsweise in der oben angegebenen Literaturstelle von Meier und Olson beschrieben. Denkbar sind für die vorliegende Erfindung weiterhin titanhaltige Zeolithe mit der Struktur des UTD-1, CIT-1, CIT-5, ZSM-48, MCM-48, ZSM-12, Ferrierit oder β-Zeolith und des Mordenits. Derartige Zeolithe sind unter anderem in der US-A 5 430 000 und der WO 94/29408 beschrieben, deren diesbezüglicher Inhalt voll umfänglich in die vorliegende Anmeldung durch Bezugnahme aufgenommen wird.

Auch bezüglich der Porenstruktur der erfindungsgemäß verwendeten Katalysatoren existieren keine besonderen Beschränkungen, d.h. der Katalysator kann Mikroporen, Mesoporen, Makroporen, Mikro- und Mesoporen, Mikro- und Makroporen oder Mikro-, Meso- und Makroporen aufweisen, wobei die Definition der Begriffe "Mesoporen" und "Makroporen" ebenfalls derjenigen in oben erwähnter Literatur gemäß Pure Appl. Chem. entspricht und Poren mit einem Durchmesser von > 2 nm bis ca. 50 nm bzw. > ungefähr 50 nm bezeichnet.

Ferner kann es sich bei dem erfindungsgemäß verwendeten Katalysator um ein Material auf der Basis eines mesoporösen mindestens ein Übergangsmetall und Silizium enthaltenden Oxids sowie eines ein Übergangsmetall und Silizium enthaltenden Xerogels handeln.

Besonders bevorzugt sind siliziumhaltige mesoporöse Oxide, die noch Ti, V, Zr, Sn, Cr, Nb oder Fe, insbesondere Ti, V, Zr, Cr, Nb oder ein Gemisch aus zwei oder mehr davon, enthalten.

Insbesondere bevorzugt werden im Rahmen des erfindungsgemäßen Verfahrens, sofern niedermolekulare Olefine, wie z.B. Propylen, umgesetzt werden, titanhaltige Zeolith-Katalysatoren eingesetzt, die ausschließlich oder nahezu ausschließlich Mikroporen aufweisen, wie z.B. Titansilikalit-1, Titansilikalit-2 oder titanhaltiger Zeolith-β, weiter bevorzugt Titansilikalit-1 oder Titans ilikalit-2, insbesondere Titansilikalit-1.

Sofern die Umsetzung gemäß Stufe (II) als Festbettverfahren durchgeführt wird, wird vorzugsweise ein mechanisch besonders stabiler Katalysator eingesetzt. Dafür kommen insbesondere Katalysatoren mit Zeolith-Struktur, wie sie in der DE-A 196 23 611 beschrieben sind, die hiermit bzgl. der darin beschriebenen Katalysatoren voll umfänglich in den Kontext der vorliegenden Anmeldung durch Bezugnahme aufgenommen wird, in Betracht.

Dabei handelt es sich um Katalysatoren auf der Basis von Titan- oder Vanadiumsilikaten mit Zeolith-Struktur, wobei bzgl. der Zeolith-Struktur auf die vorstehend als bevorzugt angegebenen Strukturen verwiesen wird. Diese Katalysatoren sind dadurch gekennzeichnet, daß sie durch verfestigende Formgebungsprozesse geformt worden sind.

Als verfestigende Formgebungsprozesse können im Prinzip alle Methoden zur einer entsprechenden Formung verwendet werden, wie sie bei Katalysatoren allgemein üblich sind. Bevorzugt werden Verfahren, bei denen die Formgebung durch Extrusion in üblichen Extrudern, beispielsweise zu Strängen mit einem Durchmesser von üblicherweise 1 bis 10 mm, insbesondere 2 bis 5 mm, erfolgt. Werden Bindemittel und/oder Hilfsmittel benötigt, ist der Extrusion zweckmäßigerweise ein Mischungs- oder Knetprozeß vorgeschaltet. Gegebenenfalls erfolgt nach der Extrusion noch ein Calcinierungsschritt. Die erhaltenen Stränge werden gewünschtenfalls zerkleinert, vorzugsweise zu Granulat oder Splitt mit einem Partikeldurchmesser von 0,5 bis 5 mm, insbesondere 0,5 bis 2 mm. Dieses Granulat oder dieser Splitt und auch auf anderem Wege erzeugte Katalysatorformkörper enthalten praktisch keine feinkörnigeren Anteile als solche mit 0,5 mm Mindestpartikeldurchmesser.

In einer bevorzugten Ausführungsform enthält der eingesetzte geformte Oxidationskatalysator bis zu 10 Gew.-% Bindemittel, bezogen auf die Gesamtmasse des Katalysators. Besonders bevorzugte Bindemittelgehalte sind 0,1 bis 7 Gew.-%, insbesondere 1 bis 15 Gew.-%. Als Bindemittel eignen sich im Prinzip alle für derartige Zwecke eingesetzte Verbindungen, bevorzugt werden Verbindungen, insbesondere Oxide, des Siliciums, Aluminiums, Bors, Phosphors, Zirkoniums und/oder Titans. Von besonderem Interesse als Bindemittel ist Siliciumdioxid, wobei das SiO₂ als Kieselsol oder in Form von Tetraalkoxysilanen in den Formgebungsschritt eingebracht werden kann. Auch als bindemittel verwendbar sind Oxide des Magnesiums und Berylliums sowie Tone, z.B. Montmorillonite, Kaoline, Bentonite, Halloysite, Dickite, Nacrite und Ananxite.

Als Hilfsmittel für die verfestigenden Formgebungsprozesse sind beispielsweise Verstrangungshilfsmittel für die Extrusion zu nennen, ein übliches Verstrangungshilfsmittel ist Methylcellulose. Derartige Mittel werden in der Regel in einem nachfolgenden Calcinierungsschritt vollständig verbrannt.

Typischerweise werden die genannten Titan- und auch Vanadiumzeolithe wie oben bei der allgemeinen Beschreibung der erfindungsgemäß verwendeten Zeolith-Katalysatoren beschrieben, hergestellt. Das so hergestellte Titan- bzw. Vanadiumzeolith-Pulver wird dann wie oben beschrieben geformt.

Ferner können Oxidationskatalysatoren auf der Basis von Titan- oder Vanadiumsilikaten mit Zeolith-Struktur mit einem Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber, die ebenfalls dadurch gekennzeichnet sind, daß sie durch verfestigende Formgebungsprozesse geformt worden sind, regeneriert werden. Derartige Katalysatoren sind in der DE-A 196 23 609.6 beschrieben, die hiermit bzgl. der darin beschriebenen Katalysatoren voll umfänglich in den Kontext der vorliegenden Anmeldung durch Bezugnahme aufgenommen wird.

Bzgl. der festigenden Formgebungsprozesse, der Bindemittel sowie der Hilfsmittel und der Struktur der Oxidationskatalysatoren trifft das oben bzgl. der DE-A 196 23 611.8 Gesagte zu.

Der in der DE-A 196 23 609.6 beschriebene Katalysator weist einen Gehalt von 0,01 bis 30 Gew.-%, insbesondere 0,05 bis 15 Gew.-%, vor allem 0,01 bis 8 Gew.-%, jeweils bezogen auf die Menge der Titan- oder Vanadium-Zeolithe, der genannten Edelmetallen auf. Hierbei wird Palladium besonders bevorzugt. Die Edelmetalle können auf den Katalysator in Form geeigneter Edelmetallkomponenten, beispielsweise in Form von wasserlöslichen Salzen, vor, während oder im Anschluß an den verfestigenden Formgebungsschritt aufgebracht werden.

In vielen Fällen ist es jedoch am günstigsten, die Edelmetallkomponenten erst nach dem Formgebungsschritt auf die Katalysatorformkörper zu bringen, besonders dann, wenn eine Hochtemperaturbehandlung des edelmetallhaltigen Katalysators unerwünscht ist. Die Edelmetallkomponenten können insbesondere durch lonenaustausch, Imprägnierung oder Aufsprühen auf den geformten Katalysator gebracht werden. Das Aufbringen kann mittels organischer Lösungsmittel, wäßriger ammoniakalischer Lösungen oder überkritischer Phasen wie etwa Kohlendioxid erfolgen.

Durch den Einsatz dieser vorgenannten Methoden können durchaus verschiedenartige edelmetallhaltige Katalysatoren erzeugt werden. So kann durch Aufsprühen der Edelmetallösung auf die Katalysatorformteile eine Art Schalenkatalysator erzeugt werden. Die Dicke dieser edelmetallhaltigen Schale läßt sich durch Imprägnieren deutlich vergrößern, während beim Ionenaustausch die Katalysatorpartikel weitgehend gleichmäßig über den Formkörperquerschnitt mit Edelmetall belegt werden.

Weiter bevorzugt wird im Rahmen des erfindungsgemäßen Verfahrens ein Zeolith-Katalysator eingesetzt, der durch ein Verfahren, das folgende Stufen umfaßt, herstellbar ist:
(i) Versetzen eines Gemischs enthaltend einen Zeolith oder ein Gemisch aus zwei oder mehr davon mit einer Mischung enthaltend mindestens einen Alkohol und Wasser, und
(ii) Kneten, Verformen, Trocknen und Calcinieren des gemäß Stufe (i) versetzten Gemischs.

Dabei werden gemäß Stufe (i) dieses Katalysator-Herstellungsverfahrens ein zeolithisches Material, vorzugsweise die oben bereits näher beschriebenen Zeolithe, insbesondere die oben bereits näher beschriebenen Titan- oder Vanadium-Zeolithe mit einer Mischung enthaltend mindestens einen Alkohol und Wasser, einem Bindemittel, gegebenenfalls einer oder mehreren organischen viskositätssteigernden Substanzen und weiteren aus dem Stand der Technik bekannten Zusatzstoffen zu einer plastischen Masse verarbeitet. Diese durch inniges Vermischen, insbesondere Kneten der obigen Komponenten erhaltene plastische Masse wird anschließend vorzugsweise durch Strangpressen oder Extrudieren verformt und der erhaltene Formkörper nachfolgend getrocknet und abschließend calciniert.

Im einzelnen läßt sich zu dem erfindungsgemäß besonders bevorzugt verwendeten Katalysator bzw. zu dessen Herstellung folgendes sagen:

Vorzugsweise handelt es sich bei dem erfindungsgemäß verwendeten Zeolith um einen Titan-, Zirkonium-, Chrom-, Niob-, Eisen- oder Vanadium-haltigen Zeolith und insbesondere ein Titansilicalit, wobei wiederum vorzugsweise ein mikroporöses Titansilicalit, weiter bevorzugt ein mikroporöses Titansilicalit mit Pentasil-Zeolith-Struktur eingesetzt wird. Dabei gilt bezüglich des Aufbaus, der Struktur, der Porenverteilung sowie der Herstellung der Zeolithe an sich das in der allgemeinen Beschreibung des erfindungsgemäß verwendeten Zeoliths Gesagte.

Als Bindemittel eignen sich im Prinzip alle für derartige Zwecke bislang eingesetzten Verbindungen. Bevorzugt werden Verbindungen, insbesondere Oxide des Siliziums, Aluminiums, Bors, Phosphors, Zirkoniums und/oder Titans verwendet. Von besonderem Interesse als Bindemittel ist Siliziumdioxid, wobei das SiO₂ als Kieselsol oder in Form von Tetraalkoxysilanen in den Formgebungsschritt eingebracht werden kann. Ferner sind Oxide des Magnesiums und Berylliums sowie Tone, z.B. Montmorillonite, Kaoline, Bentonite, Halloysite, Dickite, Nacrite und Ananxite als Bindemittel verwendbar.

Vorzugsweise wird als Bindemittel jedoch ein Metallsäureester oder ein Gemisch aus zwei oder mehr davon als Bindemittel in Stufe (I) des erfindungsgemäßen Verfahrens zugesetzt. Als solche sind insbesondere Orthokieselsäureester, Tetraalkoxysilane, Tetraalkoxytitanate, Trialkoxyaluminate, Tetraalkoxyzirkonate oder ein Gemisch aus zwei oder mehr davon zu nennen.

Besonders bevorzugt werden Tetraalkoxysilane als Bindemittel verwendet. Im einzelnen zu nennen sind dabei Tetramethoxysilan, Tetraethoxysilan, Tetrapropoxysilan und Tetrabutoxysilan, die analogen Tetraalkoxytitan- und -zirkonium-Verbindungen sowie Trimethoxy-, Triethoxy-, Tripropoxy-, Tributoxyaluminium, wobei Tetramethoxysilan und Tetraethoxysilan besonders bevorzugt sind.

Der erfindungsgemäß besonders bevorzugt verwendete Katalysator in Form eines Formkörpers enthält vorzugsweise bis zu ungefähr 80 Gew.-%, weiter bevorzugt ungefähr 1 bis ungefähr 50 Gew.-% und insbesondere ungefähr 3 bis ungefähr 30 Gew.-% Bindemittel, jeweils bezogen auf die Gesamtmasse des Formkörpers, wobei sich der Gehalt an Bindemittel aus der Menge des entstehenden Metalloxids ergibt.

Der vorzugsweise verwendete Metallsäureester wird in einer solchen Menge eingesetzt, daß der daraus entstehende Metalloxid-Gehalt im Formkörper ungefähr 1 bis ungefähr 80 Gew.-%, vorzugsweise ungefähr 2 bis ungefähr 50 Gew.-% und insbesondere ungefähr 3 bis ungefähr 30 Gew.-%, jeweils bezogen auf die Gesamtmasse des Formkörpers liegt.

Wie sich aus obigem bereits ergibt, können selbstverständlich auch Gemische aus zwei oder mehr der obengenannten Bindemittel eingesetzt werden.

Essentiell ist es, daß bei der Herstellung dieses Formkörpers als Anteigungsmittel eine Mischung enthaltend mindestens einen Alkohol und Wassers verwendet wird. Dabei beträgt der Alkoholgehalt dieser Mischung im allgemeinen ungefähr 1 bis ungefähr 80 Gew.-%, vorzugsweise ungefähr 5 bis ungefähr 70 Gew.-% und insbesondere ungefähr 10 bis ungefähr 60 Gew.-%, jeweils bezogen auf das Gesamtgewicht der Mischung.

Vorzugsweise entspricht der verwendete Alkohol der Alkoholkomponente des als Bindemittel vorzugsweise verwendeten Metallsäureesters, wobei es jedoch auch nicht kritisch ist, einen anderen Alkohol zu verwenden.

Bezüglich der verwendbaren Alkohole bestehen keinerlei Beschränkungen, sofern sie wassermischbar sind. Es können demnach sowohl Monoalkohole mit 1 bis 4 C-Atomen und wassermischbare mehrwertige Alkohole verwendet werden. Insbesondere werden Methanol, Ethanol, Propanol sowie n-, iso-, tert.-Butanol, sowie Gemische aus zwei oder mehr davon verwendet.

Als organische viskositätssteigernde Substanz können ebenfalls alle dafür geeigneten, aus dem Stand der Technik bekannten Substanzen verwendet werden. Vorzugsweise sind dies organische, insbesondere hydrophile Polymere, wie z.B. Cellulose, Stärke, Polyacrylate, Polymethacrylate, Polyvinylalkohol, Polyvinylpyrrolidon, Polyisobuten, Polytetrahydrofuran. Diese Substanzen fördern in erster Linie die Bildung einer plastischen Masse während des Knet-, Verformungs- und Trocknungsschritts durch Verbrücken der Primärpartikel und gewährleisten darüber hinaus die mechanische Stabilität des Formkörpers beim Verformen und Trocknen. Diese Substanzen werden beim Calcinieren wieder aus dem Formkörper entfernt.

Als weitere Zusatzstoffe können Amine oder aminartige Verbindungen, wie z.B. Tetraalkylammoniumverbindungen oder Aminoalkohole, sowie carbonathältige Substanzen, wie z.B. Calciumcarbonat, zugesetzt werden. Derartige weitere Zusatzstoffe sind in EP-A 0 389 041, EP-A 0 200 260 und in WO 95/19222 beschrieben, die diesbezüglich vollumfänglich in den Kontext der vorliegenden Anmeldung durch Bezugnahme einbezogen werden.

Statt basischer Zusatzstoffe ist es auch möglich saure Zusatzstoffe zu verwenden. Diese können unter anderem eine schnellere Reaktion des Metallsäureesters mit dem porösen oxidischen Material bewirken. Bevorzugt sind organische saure Verbindungen, die sich nach dem Verformungsschritt durch Calcinieren herausbrennen lassen. Besonders bevorzugt sind Carbonsäuren. Selbstverständlich können auch Gemische aus zwei oder mehr der oben genannten Zusatzstoffe eingebaut werden.

Die Zugabereihenfolge der Bestandteile der das poröse oxidische Material enthaltenden Masse ist nicht kritisch. Es ist sowohl möglich, zuerst das Bindemittel zuzugeben, anschließend die organische viskositätssteigernde Substanz, ggf. den Zusatzstoff und zum Schluß die Mischung enthaltend mindestens einen Alkohol und Wassers, als auch die Reihenfolge bezüglich des Bindemittels, der organischen viskositätssteigernden Substanz und der Zusatzstoffe zu vertauschen.

Nach der Zugabe des Bindemittels zum pulverförmigen porösen Oxid, dem gegebenenfalls die organische viskositätssteigernde Substanz bereits zugegeben worden ist, wird die in der Regel noch pulverförmige Masse 10 bis 180 Minuten im Kneter oder Extruder homogenisiert. Dabei wird in der Regel bei Temperaturen im Bereich von ungefähr 10 °C bis zum Siedepunkt des Anteigungsmittel und Normaldruck oder leichtem überathmosphärischem Druck gearbeitet. Danach erfolgt die Zugabe der restlichen Bestandteile, und das so erhaltene Gemisch wird solange geknetet, bis eine verstrangbare oder extrudierfähige, plastische Masse entstanden ist.

Prinzipiell können für die Knetung und die Verformung alle herkömmlichen Knet- und Verformungsvorrichtungen bzw. Verfahren, wie sie zahlreich aus dem Stand der Technik bekannt sind und für die Herstellung von z.B. Katalysator-Formkörpern allgemein verwendet werden.

Wie bereits angedeutet, sind jedoch Verfahren bevorzugt, bei denen die Verformung durch Extrusion in üblichen Extrudern, beispielsweise zu Strängen mit einem Durchmesser von üblicherweise ungefähr 1 bis ungefähr 10 mm, insbesondere ungefähr 2 bis ungefähr 5 mm, erfolgt. Derartige Extrusionsvorrichtungen werden beispielsweise in Ullmanns Enzyklopädie der Technischen Chemie, 4. Auflage, Bd. 2, S. 295 ff., 1972 beschrieben. Neben der Verwendung eines Extruders wird ebenfalls vorzugsweise eine Strangpresse zur Verformung verwendet.

Nach Beendigung des Strangpressens oder Extrudierens werden die erhaltenen Formkörper bei im allgemeinen ungefähr 30 °C bis 140 °C (1 bis 20 h, Normaldruck) getrocknet und bei ungefähr 400 °C bis ungefähr 800 °C (3 bis 10 h, Normaldruck) calciniert.

Selbstverständlich können die erhaltenen Stränge bzw. Extrudate zerkleinert werden. Sie werden dabei vorzugsweise zu einem Granulat oder Splitt mit einem Partikeldurchmesser von 0,1 bis 5 mm, insbesondere 0,5 bis 2 mm zerkleinert.

Dieses Granulat oder dieser Splitt und auch auf anderem Wege erzeugte Formkörper enthalten praktisch keine feinkörnigeren Anteile als solche mit ungefähr 0,1 mm Mindestpartikeldurchmesser.

Obwohl bezüglich der verwendeten Vorrichtung zur Durchführung der Umsetzung keine besonderen Beschränkungen existieren, wird Stufe (II) des erfindungsgemäßen Verfahrens vorzugsweise in einer mit einem der erfindungsgemäß verwendbaren Katalysatoren befüllten Reaktorkaskade, bestehend aus zwei bis sieben, vorzugsweise aus zwei bis fünf Reaktoren durchgeführt, wobei sich der Katalysator in Form einer Tablette oder eines Strangs als Festbett oder in Form eines Pulvers als Suspension befindet, durchgeführt. Als Beispiele für verwendbare Reaktortypen sind zu nennen: Rührkessel- und Rohrreaktoren mit oder ohne externen Umlauf.

Dabei wird ein Hydroperoxid-, vorzugsweise Wasserstoffperoxid-haltiger Strom mit einer mindestens eine C-C-Doppelbindung aufweisenden organischen Verbindung, vorzugsweise einem C₂-C₄-Olefin, weiter bevorzugt Propylen, mit einem organischen Lösungsmittel, vorzugsweise einem C₁-C₆-Alkohol, besonders bevorzugt Methanol, in Kontakt gebracht und bei ungefähr 20 °C bis ungefähr 120 °C, vorzugsweise ungefähr 30 bis ungefähr 80 °C, zur gewünschten oxidierten Verbindung, vorzugsweise zum Epoxid, umgewandelt. Bei dem vorzugsweise verwendeten Lösungsmittel Methanol kann es sich um frisches oder um von der Epoxidation zurückerhaltenes Methanol handeln.

Das Verhältnis zwischen der umzusetzenden Verbindung und dem Hydroperoxid ist nicht kritisch und liegt bei einem molaren Verhältnis von ungefähr 100:1 und 1: ungefähr 10, vorzugsweise ungefähr 1:1 bis ungefähr 6:1.

Der Gehalt an Hydroperoxid im Reaktor (ohne umzusetzende Verbindung) liegt im allgemeinen bei ungefähr 0,1 bis ungefähr 10 %, der Gehalt an Methanol liegt bei ungefähr 10 bis 90 %, und der Gehalt an Wasser liegt zwischen ungefähr 5 und ungefähr 50 %.

Auch die Menge des im Reaktor befindlichen Katalysators kann in weiten Grenzen variiert werden. Es sollte genügend Katalysator vorhanden sein, um die gewünschte Umsetzung in einer kurzen Zeitspanne zu bewältigen. Die optimale Menge hängt von vielen Faktoren wie Temperatur, Verhältnis zwischen umzusetzender Verbindung und Hydroperoxid, Reaktivität der umzusetzenden Verbindung, Reaktionsdruck, Verweilzeit und Strömungsgeschwindigkeiten der im Reaktor eingebrachten Verbindungen ab. Die Reaktionstemperatur liegt dabei im allgemeinen in einem Bereich von ungefähr 20 °C bis ungefähr 120 °C, vorzugsweise ungefähr 30 °C bis ungefähr 100 °C, weiter bevorzugt ungefähr 30 °C bis ungefähr 80 °C. Sie sollte im allgemeinen so gewählt werden, daß die gewünschte Umsetzung in einer wirtschaftlich vernünftigen Zeit durchgeführt werden kann. Die Verweilzeit liegt dabei im allgemeinen in einem Bereich von ungefähr 10 min bis ungefähr 24 h, vorzugsweise zwischen ungefähr 10 min und ungefähr 1 h pro Reaktor. Der Reaktionsdruck wird im allgemeinen in einem Bereich von ungefähr 1 bis ungefähr 100 bar, vorzugsweise ungefähr 15 bis ungefähr 40 bar, gewählt. Vorzugsweise liegt das Reaktionsgemisch in flüssiger Form vor. Die Reaktionstemperatur, Verweilzeit und Reaktionsdruck sollten so gewählt werden, daß eine Hydroperoxid-Umwandlung von mindestens 50 %, vorzugsweise von mindestens 90 % und insbesondere von 99 % oder mehr erfolgt.

Nach Beendigung der Umsetzung kann das gebildete Oxidationsprodukt vom Wasser, dem Lösungsmittel und gegebenenfalls gebildeten Nebenprodukten abgetrennt werden. Die Abtrennung kann durch alle im Stand der Technik bekannten Abtrennverfahren erfolgen, wobei destillative Abtrennverfahren bevorzugt sind.

Ein gegebenenfalls vorhandener Anteil an nicht umgesetzter, mindestens eine C-C-Doppelbindung aufweisender organischer Verbindung sowie das anfallende Lösungsmittel können ebenfalls abgetrennt und - sofern erwünscht - in die Umsetzung gemäß Stufe (II) rückgeführt werden.

Die Umsetzung gemäß Stufe (II) kann kontinuierlich, diskontinuierlich oder teilweise kontinuierlich, je nach Art der verwendeten Umsetzungsvorrichtung, wie z.B. einem Festbett, Transportbett, Flüssigbett oder auch als Suspensionsverfahren, gerührt oder nicht-gerührt, durchgeführt werden. Ferner ist eine Umsetzung in einem Einphasen- oder Mehrphasen-System, wie z.B. einem Zwei-Phasen-System, möglich. Vorzugsweise wird diese Umsetzung als Festbettverfahren durchgeführt.

Sobald die Epoxidation bis zu einem gewissen Grad durchgeführt wurde, kann das gewünschte Oxidationsprodukt vom Umsetzungsgemisch abgetrennt werden, wobei auch hier jedes aus dem Stand der Technik bekannte Abtrennverfahren, das in der Lage ist, das Oxidationsprodukt vom Umsetzungsgemisch abzutrennen, verwendet werden kann. Vorzugsweise werden destillative Abtrennverfahren verwendet.

Insbesondere bei der Durchführung der Umsetzung als Festbettverfahren fällt das erhaltene Oxidationprodukt im wesentlichen frei vom verwendeten Katalysator an und kann demgemäß ohne weitere Katalysatorabtrennungsschritte weiter aufgearbeitet werden.

In gleicher Weise kann selbstverständlich nicht umgesetztes Edukt, d.h. die mindestens eine C-C-Doppelbindung aufweisende organische Verbindung oder das Gemisch aus zwei oder mehr davon sowie das nicht umgesetzte Hydroperoxid abgetrennt und rückgeführt werden, oder aber in Spaltprodukte, wie z.B. Wasser oder Alkohol und Sauerstoff, überführt werden.

In bestimmten Ausführungsformen der vorliegenden Erfindung, insbesondere, wenn das Hydroperoxid ausgehend von einem sekundären Alkohol hergestellt wird, und somit das zur Oxidation eingesetzte, Hydroperoxid-enthaltende Gemisch auch einen sekundären Alkohol bzw. das dazu korrespondierende Keton enthält, kann letzteres durch einen Hydrierungsschritt wiederum in den sekundären Alkohol umgewandelt werden und in die Epoxidation gemäß Stufe (I) rückgeführt werden. Hydrierungsreaktionen dieser Art sind aus dem Stand der Technik wohlbekannt, wobei vorzugsweise die Hydrierung an einem Übergangsmetall-Katalysator, der beispielsweise Raney-Nickel, Ruthenium oder Palladium enthält, durchgeführt werden kann.

Auch der gegebenenfalls vorhandene sekundäre Alkohol kann unter Verwendung bekannter Methoden dehydriert werden, wobei zusätzliche Wertprodukte, wie z.B. Styrol, erhalten werden können.

### Stufe (III)

Stufe (III) des erfindungsgemäßen Verfahrens betrifft die Regenerierung des in Stufe (II) verwendeten, zumindest teilweise deaktivierten Zeolith-Katalysators.

Mit zunehmender Reaktionszeit sinkt die Aktivität des Katalysators durch zunehmende Beläge, die meist organischen Ursprungs sind. Diese, insbesondere organischen, Beläge können u.a. Oligomere oder Polymere des gebildeten Oxidationsprodukts, z.B. Propylenoxid, sein. Dabei wird das erfindungsgemäße Verfahren so durchgeführt, daß der Katalysator regeneriert wird, sofern seine Aktivität unter einen bestimmten Schwellenwert sinkt. Dieser Schwellenwert entspricht im allgemeinen einer Aktivität von 60 % oder darunter, vorzugsweise 40 % oder darunter und insbesondere 20 % oder darunter, jeweils bezogen auf die Ausgangsaktivität des zu regenerierenden Katalysators.

Sofern das erfindungsgemäße Verfahren in Suspensionsfahrweise, d.h. unter Verwendung eines Zeolith-Katalysators in Pulverform, durchgeführt wird, kann dieser Katalysator zur Regenerierung vom Reaktionsgemisch durch übliche Fest-Flüssig-Trennverfahren, wie z.B. eine einfache Filtration, Querstromfiltration, Zentrifugation, usw. abgetrennt werden. Dabei wird die Regenerierung vorzugsweise so durchgeführt, daß der sich in der Umsetzungsvorrichtung befindliche Katalysator kontinuierlich abgetrennt, regeneriert und in regenerierter Form wieder dem Reaktor zugeführt wird.

Ist der Zeolith-Katalysator in Form eines Festbetts in der Umsetzungsvorrichtung gepackt, so erfolgt die Regenerierung vorzugsweise in der Umsetzungsvorrichtung an sich, d.h. der Katalysator wird nicht ausgebaut, sondern verbleibt weiterhin gepackt im Festbett innerhalb der Umsetzungsvorrichtung.

Um sich auf dem Katalysator befindliches Wertprodukt zu gewinnen, kann der Katalysator nach der Umsetzung gemäß Stufe (II) und vor der Regenerierung gemäß Stufe (III) noch mit einem Lösungsmittel für das erhaltene Wertprodukt gewaschen werden. Als Lösungsmittel zum Waschen eignen sich alle Lösungsmittel, die in der Lage sind, das jeweils gewünschte Wertprodukt zu lösen. Insbesondere zu nennen sind dabei Wasser, Alkohole, Aldehyde, Ketone, Ether, Säuren, Ester, Nitrile und Kohlenwasserstoffe, sowie Gemische aus zwei oder mehr davon, wie sie im folgenden bei der Diskussion der bevorzugten Variante zur Durchführung der Regenerierung im Rahmen des erfindungsgemäßen Verfahrens noch diskutiert werden.

Ganz allgemein wird der Katalysator zur Regenerierung anschließend entweder in der Umsetzungsvorrichtung oder getrennt davon in einem Inertgasstrom aufgeheizt. Ab einer bestimmten Temperatur wird dem Strom von Inertgas Sauerstoff zugeführt. Diese Temperatur beträgt im allgemeinen ungefähr 200 bis ungefähr 800 °C, vorzugsweise ungefähr 250 bis 600 °C und weiter bevorzugt ungefähr mehr als 400 bis ungefähr 600 °C. Die dem Inertgas zugesetzte Sauerstoffmenge wird so eingestellt, daß die Temperatur bei der Regenerierung, die durch die Wärmeentwicklung durch den Abbrand der meist organischen Beläge ansteigt, ungefähr 800 °C, vorzugsweise ungefähr 600 °C, weiter bevorzugt ungefähr 550 °C nicht überschreitet und ungefähr 400 °C, vorzugsweise ungefähr 450 °C nicht unterschreitet, sodaß die Regenerierung einerseits mit ausreichender Schnelligkeit abläuft und andererseits irreversible Schädigungen des Katalysatorgerüsts verhindert werden.

Nach dem vollständigen Entfernen der deaktivierenden, meist organischen Beläge, was sich durch ein Absinken der Katalysatortemperatur trotz steigendem Sauerstoffgehalt am Ausgang der Regenerierungsvorrichtung bemerkbar macht, wird der Katalysator wiederum unter Inertgas langsam abgekühlt.

Wie oben ausgeführt, wird die Regenerierung gemäß Stufe (III) in einer Inertgasatmosphäre, die Sauerstoff oder eine Sauerstoff liefernde Substanz enthält, durchgeführt, wobei der Begriff "Sauerstoff liefernde Substanz" alle Substanzen umfaßt, die in der Lage sind, unter den angegebenen Regenerierungsbedingungen Sauerstoff abzugeben oder kohlenstoffhaltige Rückstände zu entfernen. Vorzugsweise handelt es sich dabei um eine stickstoffhaltige Atmosphäre mit Sauerstoff oder Sauerstoff-liefernden Substanzen. Vorzugsweise handelt es sich bei der Sauerstoff-liefernden Substanz um ein Stickoxid der Formel NₓO_{y}, wobei x und y so gewählt werden, daß sich ein neutrales Stickoxid ergibt, N₂O, einen N₂O-haltigen Abgasstrom aus einer Adipinsäureanlage, NO, NO₂, Ozon oder ein Gemisch aus zwei oder mehr davon.

Bei der Verwendung von CO₂ wird bei einer Temperatur im Bereich von 500 bis 800 °C gearbeitet.

Der Gehalt an Sauerstoff in dem zur Regenerierung verwendeten Gasgemisch beträgt vorzugsweise weniger als ungefähr 50 Vol.-%, weiter bevorzugt weniger als ungefähr 30 Vol.-%, insbesondere weniger als ungefähr 10 Vol.-% und ganz besonders bevorzugt weniger als ungefähr 5 Vol.-%.

In einer weiteren Ausführungsform des erfindungsgemäßen Verfahrens kann während des langsamen Abkühlens des regenerierten Katalysators beim Unterschreiten einer Temperatur von ungefähr 200 °C, vorzugsweise ungefähr 150 °C, weiter bevorzugt ungefähr 100 °C, der Gasstrom mit Wasser- oder einem Lösungsmitteldampf befeuchtet werden. Die dafür verwendbaren Lösungsmittel entsprechen den oben genannten Lösungsmitteln, die auch für das Waschen des zumindest teilweise deaktivierten Katalysators vor der eigentlichen Regenerierung verwendet werden können. Bevorzugte Lösungsmittel werden nachfolgend bei der Diskussion der vorzugsweise durchgeführten Regenerierung gemäß Stufe (III) des erfindungsgemäßen Verfahrens noch näher diskutiert.

Nach dem Erreichen der Umsetzungstemperatur, bei der Stufe (II) durchgeführt wird, und gegebenenfalls ausreichender Befeuchtung durch Lösungsmittel wird der regenerierte Katalysator in die Umsetzungsvorrichtung eingebracht und diese mit dem Lösungsmittel für die Oxidation befüllt und erneut für die Umsetzung gemäß Stufe (II) verwendet. Sofern der Katalysator als Festbett während der Regenerierung in der Umsetzungsvorrichtung verbleibt, wird selbstverständlich dann diese mit dem Lösungsmittel für die Oxidation befüllt und die Umsetzung gemäß Stufe (II) durchgeführt.

Im folgenden soll eine bevorzugte Ausführungsform der Regenerierung eines zumindest teilweise deaktivierten Zeolith-Katalysators gemäß Stufe (III) ausführlich diskutiert werden.

Gemäß dieser Ausführungsform wird die Regenerierung gemäß Stufe (III) wie folgt durchgeführt:
(a) Aufheizen eines zumindest teilweise deaktivierten Katalysators auf eine Temperatur im Bereich 250 °C bis 600 °C in einer Atmosphäre, die weniger als 2 Vol.-% Sauerstoff enthält, und
(b) Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von 0,1 bis 4 Vol.-% aufweist.
   Vorzugsweise umfaßt diese bevorzugte Regenerierung eine weitere Stufe (c):
(c) Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von mehr als 4 bis 100 Vol.-% aufweist.
Sowohl bei der Regenerierung von Katalysatoren in Pulverform, die als Suspension verwendet wurden, als auch bei in einem Festbett gepackten Katalysatoren in Form eines Formkörpers sowie bei der Regenerierung von auf Netze, wie z.B. ein Edelstahl, Kantal, oder Packungen kristallisierte Katalysatoren und Schalenkatalysatoren, bestehend aus inertem Kern aus SiO₂, α-Al₂O₃, hochcalciniertem TiO₂, Steatit und einer aktiven Katalysatorhülle, die ein Zeolith, vorzugsweise ein Zeolith wie oben definiert, umfaßt, verläuft diese Regenerierung im Wesentlichen identisch.

Sofern der Katalysator in Suspensionsfahrweise verwendet wurde, muß er zunächst durch einen Abtrennschritt, wie z.B. Filtration oder Zentrifugieren von der Reaktionslösung abgetrennt werden. Der so gewonnene, zumindest teilweise deaktivierte pulverförmige Katalysator kann dann der Regenerierung zugeführt werden. Die während des Regenerierungsverfahrens bei erhöhten Temperaturen durchgeführten Stufen werden bei derartigen pulverförmigen Katalysatoren vorzugsweise in Drehrohröfen durchgeführt. Bei der Regenerierung eines Katalysators, der in Suspensionsfahrweise verwendet wird, ist es besonderes bevorzugt im Rahmen einer Kopplung der Umsetzung in Suspensionsfahrweise und des erfindungsgemäßen Regenerierungsverfahrens kontinuierlich einen Teil des zumindest teilweise deaktivierten Katalysators aus der Umsetzung zu entfernen, extern mittels des erfindungsgemäßen Verfahrens zu regenerieren und den regenerierten Katalysator wieder in die Umsetzung in Suspensionsfahrweise einzuschleusen.

Neben der Regenerierung von Katalysatoren in Pulverform können auch Katalysatoren als Formkörper, beispielsweise solche, die in einem Festbett gepackt sind, regeneriert werden. Bei der Regenerierung eines im Festbett gepackten Katalysators erfolgt die Regenerierung vorzugsweise in der Umsetzungsvorrichtung selbst, wobei der Katalysator dazu weder aus- noch eingebaut werden muß, so daß er keinerlei zusätzlicher mechanischer Belastung unterliegt. Bei der Regenerierung des Katalysators in der Umsetzungsvorrichtung an sich wird zunächst die Umsetzung unterbrochen, gegebenenfalls vorhandenes Umsetzungsgemisch entfernt, die Regenerierung durchgeführt und anschließend die Umsetzung fortgesetzt.

Gemäß Stufe (a) wird der Katalysator entweder in der Umsetzungsvorrichtung oder in einem externen Ofen in einer Atmosphäre, die weniger als 2 Vol.-%, vorzugsweise weniger als 0,5 Vol.-% und insbesondere weniger als 0,2 Vol.-% Sauerstoff enthält auf eine Temperatur im Bereich von ungefähr 250 °C bis ungefähr 600 °C, vorzugsweise ungefähr 400 °C bis 550 °C und insbesondere ungefähr 450 °C bis 500 °C aufgeheizt. Dabei wird das Aufheizen gemäß Stufe (a) vorzugsweise mit einer Aufheizrate von ungefähr 0,1 °C/min. bis ungefähr 20 °C/min., vorzugsweise ungefähr 0,3 °C/min. bis ungefähr 15 °C/min. und insbesondere 0,5 °C/min. bis 10 °C/min. durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und nicht derart ansteigt, daß es zu Schädigungen der Katalysatorstruktur kommt.

Nach dem Erreichen des für die Zersetzung der Beläge gewünschten Temperaturbereichs von ungefähr 250 °C bis ungefähr 800 °C, vorzugsweise ungefähr 350 °C bis ungefähr 600 °C und insbesondere ungefähr 400 °C bis ungefähr 600 °C kann - sofern dies erwünscht, oder beim Vorliegen einer großen Menge an organischen Belägen notwendig ist - der Katalysator gegebenenfalls weitere 1 bis 2 Stunden bei diesen Temperaturen in der oben definierten Atmosphäre belassen werden.

In Stufe (a) der Regenerierung, gegebenenfalls zusammen mit dem Belassen des Katalysators bei der angegebenen Temperatur, wird der Großteil der Beläge verkokt. Die dabei gebildeten Substanzen, wie z. B. Wasserstoff, Wasser, kohlenstoffhaltige Substanzen werden im Rahmen dieser Stufe vom Katalysator entfernt. Die im Rahmen dieser Stufe betriebene Entfernung der Beläge durch Verkoken vermindert in signifikantem Maße die während des Abbrennens des Katalysators im Rahmen der Stufen (b) und ggf. (c) des erfindungsgemäßen Verfahrens durch Beaufschlagen des Katalysators mit einem Gasstrom, der einen höheren Gehalt an Sauerstoff enthält, freiwerdende Energiemenge, so daß bereits durch das langsame Aufheizen gemäß Stufe (a) des erfindungsgemäßen Verfahrens ein wesentlicher Schritt zur Verhinderung einer lokalen Überhitzung des Katalysators erreicht wird.

Gemäß Stufe (b) dieser Regenerierung wird der Katalysator anschließend bei einer Temperatur im Bereich von ungefähr 250 °C bis ungefähr 800 °C, vorzugsweise ungefähr 350 °C bis ungefähr 600 °C mit einem Gasstrom, der einen Gehalt an einer Sauerstoff-liefernden Substanz oder an Sauerstoff oder an einem Gemisch aus zwei oder mehr davon im Bereich von ungefähr 0,1 bis ungefähr 4 Vol.-%, vorzugsweise ungefähr 0,1 bis ungefähr 3 Vol.-%, weiter bevorzugt ungefähr 0,1 bis ungefähr 2 Vol.-% aufweist, beaufschlagt.

Dabei ist die zugesetzte Menge an molekularem Sauerstoff oder Sauerstoff-liefernden Substanzen insoweit kritisch, als daß mit der innerhalb dieser Stufe freiwerdenden Energiemenge, die durch den Abbrand der verkokten organischen Beläge entsteht, eine Erhöhung der Temperatur des Katalysators einhergeht, so daß die Temperatur in der Vorrichtung zur Regenerierung den gewünschten Temperaturbereich von ungefähr 250 °C bis ungefähr 800 °C, vorzugsweise ungefähr 350 °C bis ungefähr 600 °C nicht verlassen darf. Vorzugsweise wird die Menge an molekularem Sauerstoff oder Sauerstoffliefernden Substanzen so gewählt, daß die Temperatur in der Vorrichtung sich zwischen ungefähr 400 °C und ungefähr 500 °C befindet.

Mit zunehmendem Abbrand der Beläge muß der Gehalt an molekularem Sauerstoff oder Sauerstoff-liefernden Substanzen im Inertgasstrom bis hin zu 100 Vol.-% gesteigert werden, um die zur Regenerierung erforderliche Temperatur aufrecht zu erhalten. Demgemäß wird nach Beendigung der Stufe (b) im Rahmen der Stufe (c) der Katalysator im bereits bezüglich der Stufe (b) definierten Temperaturbereich mit einem Gasstrom beaufschlagt, der einen Gehalt an einer Sauerstoff-liefernden Substanz oder an Sauerstoff oder an einem Gemisch aus zwei oder mehr davon im Bereich von mehr als ungefähr 4 bis 100 Vol.-%, vorzugsweise mehr als ungefähr 3 Vol.-% bis ungefähr 20 Vol.-%, weiter bevorzugt ungefähr 2 Vol.-% bis ungefähr 20 Vol.-% aufweist.

Dabei wird in der Regel so vorgegangen, daß bei einem Absinken der Temperatur im Rahmen der Stufe (b) die Menge an Sauerstoff bzw. Sauerstoff-liefernder Substanz im zugeführten Gasstrom kontinuierlich erhöht wird.

Die Temperatur des Katalysators an sich wird durch entsprechende Steuerung des Sauerstoff-Gehalts bzw. des Gehalts an Sauerstoff-liefernden Substanzen im Gasstrom bei einer Temperatur im Bereich von ungefähr 250 °C bis ungefähr 800 °C, vorzugsweise ungefähr 350 °C bis ungefähr 600 °C, insbesondere ungefähr 400 °C bis ungefähr 600 °C gehalten.

Sinkt die Temperatur des Abgasstrom am Reaktorausgang trotz steigender Mengen an molekularem Sauerstoff oder Sauerstoff-liefernden Substanzen im Gasstrom, so ist das Abbrennen der organischen Beläge beendet. Die Dauer der Behandlung gemäß der Stufe (b) sowie ggf. der Stufe (c) beträgt im allgemeinen jeweils ungefähr 1 bis ungefähr 30, vorzugsweise ungefähr 2 bis ungefähr 20 und insbesondere ungefähr 3 bis ungefähr 10 Stunden.

Der Begriff "Sauerstoff-liefernde Substanzen" ist wie oben ausgeführt definiert.

In einer weiteren Ausführungsform des erfindungsgemäßen Verfahrens wird der zumindest teilweise deaktivierte Katalysator vor dem Aufheizen gemäß Stufe (a) mit einem Lösungsmittel gewaschen, um noch anhaftendes Wertprodukt zu entfernen. Dabei wird das Waschen so durchgeführt, daß zwar die jeweils am Katalysator anhaftenden Wertprodukte von diesem entfernt werden können, aber Temperatur und Druck nicht so hoch gewählt werden, daß die meist organischen Beläge ebenfalls entfernt werden. Vorzugsweise wird der Katalysator dabei mit einem geeigneten Lösungsmittel lediglich gespült.

Somit eignen sich für diesen Waschvorgang alle Lösungsmittel, in denen sich das jeweilige Umsetzungsprodukt gut löst. Vorzugsweise werden derartige Lösungsmittel ausgewählt aus der Gruppe bestehend aus Wasser, einem Alkohol, wie z. B. Methanol, Ethanol, 1-Propanol, 2-Propanol, 2-Methyl-2-propanol, 1-Butanol, 2-Butanol, Allylalkohol oder Ethylenglycol, einem Aldehyd, wie z. B. Acet- oder Propionaldehyd, einem Keton, wie z.B. Aceton, 2-Butanon, 2-Methyl-3-butanon, 2-Pentanon, 3-Pentanon, 2-Methyl-4-pentanon oder Cyclohexanon, einem Ether wie z. B. Diethylether oder THF, einer Säure, wie z. B. Ameisensäure, Essigsäure oder Propionsäure, einem Ester, wie z. B. Methylformiat, Methylacetat, Ethylacetat, Butylacetat oder Ethylpropionat, einem Nitril, wie z. B. Acetonitril, einem Kohlenwasserstoff, wie z. B. Propan, 1-Buten, 2-Buten, Benzol, Toluol, Xylol, Trimethylbenzol, Dichlormethan, Chloroform, Tetrachlorkohlenstoff, 1,1-Dichlorethan, 1,2-Dichlorethan, 1,1,1-Trichlorethan, 1,1,2-Trichlorethan, 1,1,1,2-Tetrachlorethan, Dibromethan, Allylchlorid oder Chlorbenzol, und, soweit mischbar, Gemischen aus zwei oder mehr davon.

Bevorzugt werden Lösungsmittel, die schon bei der Umsetzung, also z.B. die Epoxidierung von Olefin unter Verwendung des zu regenerierenden Katalysators als Lösungsmittel fungieren, eingesetzt. Als solche sind beispielhaft für die Epoxidierung von Olefinen zu nennen: Wasser, Alkohole, wie z.B. Methanol, Ethanol, 1-Propanol, 2-Propanol, 2-Methyl-2-propanol, 1-Butanol, 2-Butanol, Allylalkohol oder Ethylenglycol, oder Ketone, wie z.B. Aceton, 2-Butanon, 2-Methyl-3-butanon, 2-Pentanon, 3-Pentanon, 2-Methyl-4-pentanon oder Cyclohexanon.

Die benutzte Menge an Lösungsmittel sowie die Dauer des Waschvorgangs sind nicht kritisch, sowohl Menge an Lösungsmittel als auch Dauer des Waschvorgangs sollten jedoch ausreichen, um einen Großteil des am Katalysator haftenden Wertprodukts zu entfernen. Der Waschvorgang kann bei der Temperatur der Umsetzung oder bei verglichen dazu erhöhten Temperaturen erfolgen, wobei die Temperatur jedoch nicht so hoch sein sollte, daß das zum Waschen verwendete Lösungsmittel selbst wieder mit dem zu entfernenden Wertprodukt reagiert. Sofern Temperaturen, die oberhalb der Umsetzungstemperatur liegen, verwendet werden, ist im allgemeinen ein Bereich von 5 °C bis 150 °C oberhalb der Umsetzungstemperatur, insbesondere auch bedingt durch den Siedepunkt der verwendeten Lösungsmittel, ausreichend. Der Waschvorgang kann falls erforderlich, mehrmals wiederholt werden. Der Waschvorgang kann unter Normaldruck, erhöhtem Druck oder sogar überkritischem Druck erfolgen. Bevorzugt sind Normaldruck und erhöhter Druck. Bei Verwendung von CO₂ als Lösungsmittel ist überkritischer Druck bevorzugt.

Wird ein in Suspensionsfahrweise verwendeter pulverförmiger Katalysator regeneriert, erfolgt das Waschen des abgetrennten Katalysators in einem externen Reaktor. Ist der Katalysator in Form eines Festbetts in einem Reaktor gepackt, so kann das Waschen im Umsetzungsreaktor erfolgen. Dabei wird dieser mit dem darin befindlichen zu regenerierenden Katalysator ein- oder mehrmals mit dem Lösungsmittel gespült, um das restliche Wertprodukt zu gewinnen. Anschließend wird das Lösungsmittel aus dem Reaktor entfernt.

Nach der Beendigung des Waschvorgangs wird der Katalysator im allgemeinen getrocknet. Obwohl der Trocknungsvorgang an sich nicht kritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere - falls vorhanden - den Mikroporen des Zeolith-Katalysators zu vermeiden, da auch dies zu einer Schädigung desselben führen kann. Bei Regenerierung von pulverförmigen Katalysatoren erfolgt die Trocknung wiederum extern in einer Heizvorrichtung unter Inertgasatmosphäre. Bei Katalysatoren im Festbett wird der im Reaktor befindliche Katalysator bei mäßigen Temperaturen mit einem Inertgasstrom beaufschlagt. Die Trocknung des Katalysators kann, muß aber nicht bis zur Vollständigkeit durchgeführt werden. Bei pulverförmigen Katalysatoren wird in der Regel so weit getrocknet, bis das Pulver rieselfähig ist. Auch bei Katalysatoren, die in einem Festbett eingebaut sind, ist eine vollständige Trocknung in der Regel nicht nötig.

In einer weiteren Ausführungsform dieser Regenerierung wird in einer zusätzlichen Stufe (d) der in Stufe (c) erhaltene regenerierte Katalysator in einem Inertgasstrom abgekühlt. Dieser Inertgasstrom kann bis zu 20 Vol.-%, vorzugsweise ungefähr 0,5 bis ungefähr 20 Vol.-% eines Flüssigkeitsdampfes, ausgewählt aus der Gruppe bestehend aus Wasser, einem Alkohol, einem Aldehyd, einem Keton, einem Ether, Säure, einem Ester, einem Nitril, einem Kohlenwasserstoff, wie oben bzgl. des Waschens des Katalysators beschrieben, und einem Gemisch aus zwei oder mehr davon, enthalten. Vorzugsweise werden Wasser, Alkohol, oder ein Gemisch aus zwei oder mehr davon als Flüssigkeitsdampf verwendet.

Bezüglich der vorzugsweise verwendbaren Alkohole, Aldehyde, Ketone, Ether, Säuren, Ester, Nitrile oder Kohlenwasserstoffe wird auf die entsprechende Diskussion der im Rahmen des Waschvorgangs im erfindungsgemäßen Verfahren verwendbaren Lösungsmittel verwiesen.

Dabei ist es auch bei dem Abkühlen gemäß Stufe (d) wichtig, daß langsam abgekühlt wird, da ein zu schnelles Abkühlen ("Abschrecken") die mechanische Festigkeit des Katalysators negativ beeinflussen kann. Ebenso kann die Mechanik des Katalysators durch schnelles Spülen der regenerierten, trockenen Katalysatorformkörper beim Wiederanfahren des Reaktors für die weitere Umsetzung negativ beeinflußt werden. Aus diesem Grund empfiehlt es sich, während der Abkühlphase den oben definierten Flüssigkeitsdampf zuzusetzen. Weiter bevorzugt wird dieser jedoch erst unterhalb einer sogenannten Schwellentemperatur zugesetzt, die durch die Siedetemperatur der für den Dampf verwendeten Flüssigkeit definiert wird. Die Schwellentemperatur liegt dabei in der Regel unterhalb von ungefähr 250 °C, vorzugsweise unterhalb von ungefähr 200 °C und insbesondere unterhalb von ungefähr 150 °C.

Nach der Regenerierung kann der Katalysator mit basischen und/oder silylierenden Verbindungen zur Entfernung von sauren Zentren behandelt werden. Als derartige Verbindungen eignen sich dort insbesondere verdünnte wäßrige Lösungen von Alkali- oder Erdalkalihydroxiden, -carbonaten, -hydroxycarbonaten; Li-, K-, Na-Acetate und -Phosphate; sowie als silylierende Verbindungen silylierende Ester, wie z.B. Tetraalkoxysilane, Tetraalkoxymonoalkylsilane sowie Hexamethylendisilane.

### Stufe (IV)

Diese Stufe betrifft die Wiederverwendung des gemäß Stufe (III) regenerierten Katalysators. Dazu wird - sofern der zumindest teilweise deaktivierte Katalysator extern regeneriert wurde - der regenerierte Katalysator in die Umsetzungsvorrichtung zurückgeführt und die Umsetzung wie bezüglich Stufe (II) hierin beschrieben durch- bzw. weitergeführt.

Sofern die Regenerierung innerhalb der Umsetzungsvorrichtung durchgeführt wurde, wird nach Beendigung derselben die Umsetzung wie hierin bezüglich Stufe (II) beschrieben weitergeführt.

Sofern im Rahmen des erfindungsgemäßen Verfahrens die mindestens eine C-C-Doppelbindung aufweisende organische Verbindung ausgewählt wird aus der Gruppe bestehend aus einem linearen oder verzweigtkettigen aliphatischen, einem linearen oder verzweigtkettigen aromatischen und einem linearen oder verzweigtkettigen cycloaliphatischen Olefin, mit jeweils bis zu 30 C-Atomen, d.h. sofern ein Olefin zur Umsetzung mit dem Hydroperoxid eingesetzt wird, kann dieses durch Dehydrierung der entsprechenden gesättigten organischen Verbindung unter Erhalt des Olefins und Wasserstoff erhalten werden.

Derartige Verfahren zur Umwandlung eines Alkans in das entsprechende Olefin sind an sich, insbesondere bezüglich der Propan-Dehydrierung bekannt. Sie sind in der Literatur unter der Bezeichnung STAR, CATOFIN® oder OLEFLEX® -Verfahren bekannt und z.B. in Chem. Systems Report 91-5, 1992, S. 50 ff. detailliert beschrieben, außerdem wird in zahlreichen Patenten, wie z.B. US-A 4,665,267 oder EP-A 0 328 507 sowie der US-A 4,886,928 darauf Bezug genommen.

Charakteristisch für diese Verfahren ist dabei, daß in einer endothermen Reaktion das Alkan zum Olefin, also beispielsweise Propan zu Propen, und Wasserstoff gespalten wird. Als Katalysatoren kommen dabei Zink-, Aluminium-Spinelle mit Edelmetall-Dotierung, Chromoxid/Aluminiumoxid, sowie Platin-Trägerkatalysatoren verbreitet zum Einsatz.

Ferner kennt man aus der DE-A 39 23 026 promotierte Eisenoxid-Katalysatoren zur Durchführung von Alkan-Dehydrierungen.

Ferner kann das als Ausgangsprodukt vorzugsweise verwendete Olefin, insbesondere Propylen, ausgehend vom entsprechenden gesättigten Kohlenwasserstoff durch Steam-Cracking, katalytisches Cracken erhalten werden. Weitere Details bezüglich derartiger Verfahren sind u.a. den eingangs erwähnten US-A 5,599,955 und US-A 5,599,956 und des darin zitierten Standes der Technik zu entnehmen, wobei. diese beiden Druckschriften einschließlich des darin zitierten Standes der Technik bezüglich dieses Aspekts der vorliegenden Erfindung vollumfänglich durch Bezugnahme in den Kontext der vorliegenden Anmeldung einbezogen werden.

Insbesondere bei der Durchführung des erfindungsgemäßen Verfahrens als integriertes Verfahren, d.h. als Verfahren, bei dem die Volumenströme allesamt geschlossen sind, ist es vorteilhaft, wenn man das in die Epoxidierungsstufe einzusetzende Olefin, insbesondere das Propylen, durch Dehydrierung der entsprechenden gesättigten organischen Verbindung erhält, da zum einen die Epoxidierungsstufe auch das neben dem Olefin noch vorhandene, nicht umgesetzte Alkan aus der Dehydrierungsstufe toleriert und somit eine teuere Alkan/Olefin-, insbesondere Propan/Propen-Trennung erspart.

Ferner kann der aus der Alkan-Dehydrierung stammende Wasserstoff direkt bei der Wasserstoffperoxid-Bildung, z.B. nach dem eingangs beschriebenen Anthrachinon-Verfahren oder dem Verfahren ausgehend von den Elementen, wie eingangs bei der Diskussion der Stufe (I) des erfindungsgemäßen Verfahrens beschrieben, verwendet werden.

Ferner kana der endotherme Schritt der Alkan-Dehydrierung mit der exothermen Umsetzung gemäß Stufe (II) in einem Wärme- und Energieverbund gekoppelt werden.

Wie oben bereits angedeutet, eignet sich das erfindungsgemäße Verfahren insbesondere zur Durchführung des Verfahrens als integriertes Verfahren, d.h. als mehrstufiges Verfahren, bei dem die Ströme der verschiedenen während des Verfahrens verwendeten Komponenten zum Teil oder vollständig geschlossen sind, weiter bevorzugt zusammen mit einer entsprechenden Wärme- und Energiekopplung, in der die bei den exothermen Verfahrensstufen (II) und (III) freiwerdenden Energiemengen direkt zum Betreiben der endothermen Stufe (I) verwendet werden kann.

Im folgenden soll die vorliegende Erfindung noch anhand von einigen Ausführungsbeispielen erläutert werden.

### BEISPIELE

### Beispiel 1

### Synthese von Wasserstoffperoxid gemäß dem Anthrachinon-Verfahren

600 kg einer Arbeitslösung, bestehend aus ca. 10 Gew.-% 2-Ethylanthrachinon, gelöst in einem Gemisch aus 70 Vol.-% Shellsol NF und 30 Vof.-% Tetrabutylharnstoff, wurden mit 10 kg eines Pd auf Aktivkohle-Hydrierkatalysators (10 Gew.-% Palladium) versetzt und in einem Rührkessel bei 45 °C mit Wasserstoff bei einem Druck von 1,5 bis 2 bar in Kontakt gebracht, bis der theoretische Wasserstoffverbrauch erreicht wurde. Die nun schwarzgefärbte Lösung wurde auf Raumtemperatur abgekühlt und der Katalysator durch Filtration abgetrennt. Die Hydrochinon-haltige Lösung wurde in einem Strahldüsenreaktor in drei Anteilen mit je 200 kg mit verdünnter Luft (10 Vol.-% Sauerstoff, 90 Vol.-% Stickstoff) solange oxidiert, bis der Wasserstoffperoxid-Gehalt konstant war. Nach der Oxidation wurden 200 kg des nun ca. 1 Gew.-% Wasserstoffperoxid enthaltenden Gemischs mit ca. 15 kg VE-Wasser versetzt und 15 min lang intensiv gerührt. Anschließend wurde die wäßrige Phase abgetrennt. Mit der nun ungefähr 9 Gew.-% Wasserstoffperoxid enthaltenden wäßrigen Lösung wurde der nächste 200 kg-Anteil 15 min lang intensiv gerührt. Nach der Abtrennung erhielt man ein Gemisch, das einen Anteil an Wasserstoffperoxid von ca. 15 Gew.-% aufwies, mit dem der letzte 200 kg-Anteil auf die gleiche Weise extrahiert wurde. Auf diese Weise ließen sich ungefähr 15 kg einer ungefähr 20 Gew.-% Wasserstoffperoxid enthaltenden wäßrigen Lösung gewinnen.

Bessere Ausbeuten an Wasserstoffperoxid können mit einer, kontinuierlichen Gegenstrom-Extraktion z.B. in einer Siebbodenkolonne, einer gepulsten Siebbodenkolonne und einer Füllkörperkolonne erhalten werden.

### Beispiel 2

### Synthese von Wasserstoffperoxid gemäß der DE-A 196 42 770

Bei der Herstellung von Wasserstoffperoxid nach der obengenannten Anmeldung wurde als Reaktionsgefäß ein 270 ml Autoklav mit Rührer, Thermostatisierung und Druckhaltung von 50 bar verwendet. In diesen Reaktor wurde ein wie folgt hergestellter Katalysatormonolith um die Rührerachse zentriert eingebaut, so daß er durch den Rührer gleichmäßig mit Flüssigkeit und Gas versorgt wurde. Im Reaktorboden befanden sich Zuleitungen für Sauerstoff, Wasserstoff und das Reaktionsmedium. Im Reaktordeckel befand sich eine Ableitung, aus der das Produkt/Gasgemisch kontinuierlich entnommen werden konnte. Nach Abzug der Volumina für alle Einbauten stand ein effektives Reaktionsvolumen von 208 ml zur Verfügung.

### Der hierbei verwendete Katalysatormonolith wurde wie folgt hergestellt:

Ein gewelltes und ein glattes Netz aus V4A-Stahl (1.4571, Maschenweite 180 µm, Drahtdurchmesser 146 µm) wurden aufeinander gelegt und zu einem zylinderförmigen Monolith mit einer Höhe von 5 cm und einem Durchmesser von ebenfalls 5 cm gerollt. Die Enden der Netze wurden durch Schweißpunkte fixiert. Der Netzendenabstand der glätten Netze betrug wenigstens 1 mm.

Der monolithische Träger wurde sukzessive mit Aceton und destilliertem Wasser behandelt und anschließend getrocknet. Danach wurde der Monolith mit einer Lösung aus 25 Gew.-% konzentrierter Salzsäure und 75 Gew.-% destilliertem Wasser 10 min lang bei 60 °C behandelt und mit destilliertem Wasser abgespült. Der so behandelte Monolith wurde in 150 ml destilliertem Wasser vorgelegt. Nach Zugabe von 10 Tropfen konzentrierter HNO₃ und 36 ml einer 1 gew.-%igen wäßrigen Lösung von hypophosphoriger Säure wurden 20 ml einer Palladiumnitratlösung mit einer Palladiumkonzentration von 1 Gew.-% zugegeben. Hiernach wurde zuerst 17 min auf 60 °C und dann eine Stunde lang auf 80 °C erwärmt. Anschließend ließ man abkühlen, wusch den Katalysatormonolith mit destilliertem Wasser und trocknete ihn 16 Stunden lang bei 120 °C.

Das zur Herstellung von Wasserstoffperoxid verwendete Reaktionsmedium bestand aus Methanol, dem 0,4 Gew.-% Schwefelsäure, 0,1 Gew.-% Phosphorsäure und 6 ppm Bromid (in Form von Natriumbromid) zugesetzt worden waren. Mit dem Reaktionsmedium wurde der Reaktor geflutet. Anschließend leitete man einen Strom von 72,8 g/h Reaktionsmedium, 48,6 l/h Sauerstoff und 5,5 l/h Wasserstoff (Gase bezogen auf Normalbedingungen) durch den Reaktor. Am Reaktordeckel wurde das Produkt/Gasgemisch kontinuierlich entnommen.

Der Umsatz bezogen auf Wasserstoff betrug 76 % (gemäß einer Bestimmung des Wasserstoffgehaltes im Abgas) bei einer Selektivität von 82 %. Die Konzentration der so hergestellten methanolischen Wasserstoffperoxid-Lösung lag bei 7 Gew.-% (Titration mit KMnO₄ 0,1 N).

### Beispiel 3

### Epoxidation von Propen mit Wasserstoffperoxid am Festbettkatalysator

Durch eine Reaktorkaskade von zwei Reaktoren mit je 190 ml Reaktionsvolumen, gefüllt mit je 10 g Titansilikalit-1 (TS-1), der zu Vollkontaktsträngen mit 2 mm Durchmesser verformt wurde, wurden Flüsse von 27,5 g/h Wasserstoffperoxid (20 Gew.-%, gewonnen gemäß Beispiel 1), 65 g/h Methanol und 13,7 g/h Propylen bei 40 °C Reaktionstemperatur und 20 bar Reaktionsdruck durchgeleitet. Nach Verlassen des zweiten Reaktors wurde das Reaktionsgemisch in einem Sambay-Verdampfer gegen Atmosphärendruck entspannt. Die abgetrennten Leichtsieder wurden on-line in einem Gaschromatographen analysiert. Der flüssige Reaktionsaustrag wurde gesammelt, gewogen und ebenfalls gaschromatographisch analysiert.

Während der gesamten Laufzeit sank der Wasserstoffperoxid-Umsatz von ursprünglich 96 % und erreichte nach 400 h einen Wert von 63 %. Die Selektivität bezogen auf Wasserstoffperoxid betrug 95 %.

### Beispiel 4

### Regenerierung des deaktivierten Katalysators

Der deaktivierte, mit organischen Produkten belegte, in Beispiel 3 verwendete Festbettkatalysator wurde mit Methanol gespült und anschließend bei 120 °C fünf Stunden lang getrocknet. 56 g des getrockneten verformten Katalysators wurden in ein Drehrohr eingebaut. Unter sehr langsamem Drehen des Drehrohrs (2 U/h) wurde unter Stickstoff (20 l/h) zuerst mit 4 °C/min auf 500 °C aufgeheizt. Danach wurde für 2 h bei 500 °C ein Gasgemisch (20 l/h) in das Drehrohr gefahren, das 9 Vol.-% Sauerstoff und 91 Vol.-% Stickstoff enthielt. Danach wurde für 14 h bei 500 °C und gleichbleibender Gasmenge (20 1/h) der Volumenanteil an Sauerstoff im Gasstrom auf 18 Vol.-% erhöht. Danach wurde der regenerierte Katalysator im stetigen Gasstrom abgekühlt. Der Gewichtsverlust betrug ungefähr 7 %.

### Beispiel 5

### Erneuter Einsatz von regeneriertem Katalysator

Durch eine Reaktorkaskade von zwei Reaktoren mit je 190 ml Reaktionsvolumen, gefüllt mit je 10 g des gemäß Beispiel 4 regenerierten Katalysators wurden Flüsse von 27,5 g/h Wasserstoffperoxid (20 Gew.-%, gewonnen gemäß Beispiel 1), 65 g/h Methanol und 13,7 g/h Propylen bei 40 °C Reaktionstemperatur und 20 bar Reaktionsdruck durchgeleitet. Nach Verlassen des zweiten Reaktors wurde das Reaktionsgemisch in einem Sambay-Verdampfer gegen Atmosphärendruck entspannt. Die abgetrennten Leichtsieder wurden On-line in einem Gaschromatographen analysiert. Der flüssige Reaktionsaustrag wurde gesammelt, gewogen und ebenfalls gaschromatographisch analysiert.

Während der gesamten Laufzeit sank der Wasserstoffperoxid-Umsatz von ursprünglich 96 % und erreichte nach 400 h einen Wert von 63 %. Die Selektivität bezogen auf Wasserstoffperoxid betrug 95 %.

### Beispiel 6

### Dehydrierung von Propan zu Propen

In einen Doppelmantel-Rohrreaktor (Länge 50 cm mit 35 mm Innendurchmesser) wurden 210 ml eines Dehydrierkatalysators auf Basis Cr-Oxid/Al₂O₃ in Form von 2mm-Strängen eingebaut. Mit Hilfe eines Salzbad-Wärmeträgers wurde der Reaktor auf 550 °C Wandtemperatur aufgeheizt. Propan wurde als Gemisch mit Stickstoff (Volumenverhältnis 20:80) aus einer Stahlflasche druckgeregelt bei 1,5 bar (LHSV = 0,15/h) über den Reaktor geleitet. Das austretende Reaktionsgemisch aus Propan, Propen und Wasserstoff wurde auf 30 bis 40 °C abgekühlt und zur Abtrennung der C₃-Produkte vom Wasserstoff auf ca. 35 bar zur Verflüssigung verdichtet. Dieses Flüssiggasgemisch konnte ohne weitere Aufreinigung in der Epoxidation eingesetzt werden, da dort nur das Propylen umgesetzt wurde und Propan sich hinreichend inert verhielt.

Nach der Epoxidation nichtumgesetztes C₃-Propan/Propen-Gemisch konnte nach Prüfung auf Peroxid-Freiheit entspannt und erneut in den Reaktor zur Propan-Dehydrierung zurückgefahren werden.

Nach einer Reaktionsdauer von 3 Stunden betrug der Umsatz an Propan pro Durchlauf typischerweise ca. 35% in einer Selektivität zu Propen (GC-Analyse vor dem Verdichter) von 83 mol-%.

Der desaktivierte Katalysator ließ sich wieder regenerieren, wenn man nach Schließen der Propan-Zufuhr Luft in das Stickstoffträgergas eindosierte (max. 2 Vol.-% Sauerstoff).

### Beispiel 7

### Direktsynthese von Wasserstoffperoxid in Wasser

Es wurde der gleiche Katalysator wie in Beispiel 4 verwendet. Das Reaktionsmedium bestand aus Wasser, dem 0,4 Gew.-% Schwefelsäure, 0,1 Gew.-% Phosphorsäure und 6 ppm Bromid (in Form von Natriumbromid) zugesetzt wurden. Die Reaktionsparameter waren wie folgt: 268,0 g/h Reaktionsmedium, 291,6 l/h Sauerstoff, 32,4 l/h Wasserstoff, T = 42 °C. Der Umsatz bezogen auf Wasserstoff wurde durch eine Wasserstoffbestimmung des Abgases erhalten und betrug 43% bei einer Selektivität von 70%. Die Konzentration der so hergestellten Wasserstoffperoxid-Lösung lag bei 5,6 Gew.-%.

## Patentansprüche

1. Verfahren zur Oxidation einer mindestens eine C-C-Doppelbindung aufweisenden organischen Verbindung oder eines Gemischs aus zwei oder mehr davon, das die folgenden Stufen umfaßt:
(I) Herstellung eines Hydroperoxids,
(II) Umsetzung einer mindestens eine C-C-Doppelbindung aufweisenden organischen Verbindung oder eines Gemischs aus zwei oder mehr davon mit dem in Stufe (I) hergestellten Hydroperoxid in Gegenwart eines Zeolith-Katalysators,
(III) Regenerierung des in Stufe (II) verwendeten, zumindest teilweise deaktivierten Zeolith-Katalysators, wobei die Regenerierung mindestens die folgenden Schritte aufweist:
(a) Aufheizen des Zeolith-Katalysators in einem Inertgasstrom;
(b) Zuführen von Sauerstoff bei einer Temperatur in einem Bereich von 200 °C bis 800 °C;
(c) Einstellen der zugeführten Sauerstoffmenge, so daß die Temperatur in einem Bereich von 400 °C bis 800 °C liegt.
(IV) Durchführung der Umsetzung gemäß Stufe (II) unter Verwendung eines Zeolith-Katalysators, der den in Stufe (III) regenerierten Katalysator enthält.

2. Verfahren nach Anspruch 1, wobei die mindestens eine C-C-Doppelbindung aufweisende organische Verbindung ausgewählt wird aus der Gruppe bestehend aus einem linearen oder verzweigtkettigen aliphatischen, einem linearen oder verzweigtkettigen aromatischen, einem linearen oder verzweigtkettigen cycloaliphatischen Olefin, mit jeweils bis zu 30 C-Atomen und einem Gemisch aus zwei oder mehr davon.

3. Verfahren nach Anspruch 2, wobei das Olefin erhalten wird durch Dehydrierung der entsprechenden gesättigten organischen Verbindung unter Erhalt des Olefins und Wasserstoff.

4. Verfahren nach Anspruch 3, wobei die Dehydrierung in Gegenwart eines heterogenen Katalysators durchgeführt wird, der mindestens eines der folgenden Elemente enthält:
Sc, Y, La, Ti, Zr, Hf, V, Nb, Ta, Cr, Mo, W, Mn, Re, Fe, Ru, Os, Co, Rh, Ir, Ni, Pd, Pt, Cu, Ag, Au, B, Al, Ga, C, Si, Ge und Sn.

5. Verfahren nach einem der Ansprüche 1 bis 4, wobei der Zeolith-Katalysator Mikroporen, Mesoporen, Makroporen, Mikro- und Mesoporen, Mikro- und Makroporen, oder Mikro-, Meso- und Makroporen aufweist.

6. Verfahren nach einem der Ansprüche 1 bis 5, wobei der Zeolith-Katalysator ausgewählt wird aus der Gruppe bestehend aus einem Titan-, Zirkonium-, Vanadium-, Chrom- oder Niob- enthaltenden Silikat, mit MFI-, BEA-, MOR-, TON-, MTW-, FER-, CHA-, ERI-, RHO-, GIS-, BOG-, NON-, EMT-, HEU-, KFI-, FAU-, DDR-, MTT-, RUT-, LTL-, MAZ-, GME-, NES-, OFF-, SGT-, EUO-, MFS-, MCM-22-, MEL-Struktur, MFI/MEL-Mischstruktur und einem Gemisch aus zwei oder mehr davon.

7. Verfahren nach einem der Ansprüche 1 bis 6, wobei als Zeolith-Katalysator ein Katalysator, herstellbar durch ein Verfahren, das folgende Stufen umfaßt, verwendet wird:
(i) Versetzen eines Gemischs enthaltend einen Zeolith oder ein Gemisch aus zwei oder mehr davon mit einer Mischung enthaltend mindestens einen Alkohol und Wasser, und
(ii) Kneten, Verformen, Trocknen und Calcinieren des gemäß Stufe (i) versetzten Gemischs.

8. Verfahren nach einem der Ansprüche 1 bis 7, wobei die Regenerierung des zumindest teilweise deaktivierten Zeolith-Katalysators gemäß Stufe (III) wie folgt durchgeführt wird:
(a) Aufheizen eines zumindest teilweise deaktivierten Katalysators auf eine Temperatur im Bereich 250 °C bis 600 °C in einer Atmosphäre, die weniger als 2 Vol.-% Sauerstoff enthält, und
(b) Beaufschlagen des Katalysators bei einer Temperatur im Bereich von 250 bis 800 °C, vorzugsweise 350 bis 600 °C, mit einem Gasstrom, der einen Gehalt an einer Sauerstoff-liefernden Substanz oder an Sauerstoff oder an einem Gemisch aus zwei oder mehr davon im Bereich von 0,1 bis 4 Vol.-% aufweist.

9. Verfahren nach einem der Ansprüche 1 bis 8, wobei die Regenerierung gemäß Stufe (III) des zumindest teilweise deaktivierten Katalysators in einer Vorrichtung zur Umsetzung gemäß Stufe (II) durchgeführt wird, ohne daß der Zeolith-Katalysator aus dieser Vorrichtung entfernt wird.

## Claims

1. A process for oxidizing an organic compound having at least one C-C double bond or a mixture of two or more thereof, which comprises the following steps:
(I) preparing a hydroperoxide,
(II) reacting an organic compound having at least one C-C double bond or a mixture of two or more thereof with the hydroperoxide prepared in step (I) in the presence of a zeolite catalyst,
(III) regenerating the at least partially deactivated zeolite catalyst used in step (II), the regeneration at least comprising the following steps:
(a) heating the zeolite catalyst in a stream of inert gas;
(b) adding oxygen at a temperature in a range from 200°C to 800°C;
(c) regulating the amount of oxygen added in such a manner that the temperature is in a range from 400°C to 800°C,
(IV) conducting the reaction of step (II) using a zeolite catalyst containing the catalyst regenerated in step (III).

2. A process as claimed in claim 1, wherein the organic compound having at least one C-C double bond is selected from the group consisting of a linear or branched aliphatic olefin, a linear or branched aromatic olefin, a linear or branched cycloaliphatic olefin, each having up to 30 carbon atoms, and a mixture of two or more thereof.

3. A process as claimed in claim 2, wherein the olefin is obtained by dehydrogenating the corresponding saturated organic compound to obtain the olefin and hydrogen.

4. A process as claimed in claim 3, wherein the dehydrogenation is carried out in the presence of a heterogeneous catalyst containing at least one of the following elements:
Sc, Y, La, Ti, Zr, Hf, V, Nb, Ta, Cr, Mo, W, Mn, Re, Fe, Ru, Os, Co, Rh, Ir, Ni, Pd, Pt, Cu, Ag, Au, B, Al, Ga, C, Si, Ge and Sn.

5. A process as claimed in any of claims 1 to 4, wherein the zeolite catalyst has micropores, mesopores, macropores, micro- and mesopores, micro- and macropores and micro-, meso- and macropores.

6. A process as claimed in any of claims 1 to 5, wherein the zeolite catalyst is selected from the group consisting of a silicate containing titanium, zirconium, vanadium, chromium oder niobium and having MFI, BEA, MOR, TON, MTW, FER, CHA, ERI, RHO, GIS, BOG, NON, EMT, HEU, KFI, FAU, DDR, MTT, RUT, LTL, MAZ, GME, NES, OFF, SGT, EUO, MFS, MCM-22, MEL structure, MFI/MEL mixed structure and a mixture of two or more thereof.

7. A process as claimed in any of claims 1 to 6, wherein the zeolite catalyst used is a catalyst obtainable by a process which comprises the following steps:
(i) adding to a mixture comprising a zeolite or a mixture of two or more thereof a mixture comprising at least one alcohol and water, and
(ii) kneading, shaping, drying and calcining of the mixture of step (i).

8. A process as claimed in any of claims 1 to 7, wherein the at least partially deactivated zeolite catalyst of step (III) is regenerated by the following steps:
(a) heating of an at least partially deactivated catalyst to 250°C-600°C in an atmosphere containing less than 2% by volume of oxygen, and
(b) subjecting the catalyst to a gas stream containing an oxygen-generating substance or oxygen or a mixture of two or more thereof in an amount in the range from 0.1 to 4% by volume at from 250 to 800°C, preferably at from 350 to 600°C.

9. A process as claimed in any of claims 1 to 8, wherein the regeneration of step (III) of the at least partially deactivated catalyst is carried out in an apparatus for conducting the reaction of step (II) without removing the zeolite catalyst from this apparatus.

## Revendications

1. Procédé pour l'oxydation d'un composé organique comportant au moins une double liaison C-C ou d'un mélange d'au moins deux composés de ce type, qui comporte les étapes suivantes :
(I) préparation d'un hydroperoxyde,
(II) réaction d'un composé organique comportant au moins une double liaison ou d'un mélange d'au moins deux composés de ce type avec l'hydroperoxyde préparé dans l'étape (I), en présence d'un catalyseur zéolitique,
(III) régénération du catalyseur zéolitique utilisé dans l'étape (II), au moins partiellement désactivé, la régénération comportant au moins les étapes suivantes :
(a) chauffage du catalyseur zéolitique dans un courant d'un gaz inerte,
(b) amenée d'oxygène à une température comprise dans la plage de 200 à 800°C,
(c) ajustement du débit d'amenée d'oxygène de façon que la température soit comprise dans la plage de 400 à 800°C,
(IV) mise en oeuvre de la réaction selon l'étape (II) par utilisation d'un catalyseur zéolitique contenant le catalyseur régénéré dans l'étape (III).

2. Procédé selon la revendication 1, dans lequel le composé organique comportant au moins une double liaison C-C est choisi dans l'ensemble comprenant les oléfines aliphatiques à chaîne droite ou ramifiée, les oléfines aromatiques à chaîne droite ou ramifiée, les oléfines cycloaliphatiques à chaîne droite ou ramifiée, chacune ayant jusqu'à 30 atomes de carbone, et les mélanges d'au moins deux d'entre elles.

3. Procédé selon la revendication 2, dans lequel l'oléfine est obtenue par déshydrogénation du composé organique saturé correspondant, avec obtention de l'oléfine et d'hydrogène.

4. Procédé selon la revendication 3, dans lequel la déshydrogénation est mise en oeuvre en présence d'un catalyseur hétérogène qui contient au moins l'un des éléments suivants :
Sc, Y, La, Ti, Zr, Hf, V, Nb, Ta, Cr, Mo, W, Mn, Re, Fe, Ru, Os, Co, Rh, Ir, Ni, Pd, Pt, Cu, Ag, Au, B, Al Ga, C, Si, Ge et Sn.

5. Procédé selon l'une des revendications 1 à 4, dans lequel le catalyseur zéolitique comporte des micropores, des mésopores, des macropores, des micro- et des mésopores, des micro- et des macropores, ou des micro-, des méso- et des macropores.

6. Procédé selon l'une des revendications 1 à 5, dans lequel le catalyseur zéolitique est choisi dans l'ensemble comprenant les silicates contenant du titane, du zirconium, du vanadium, du chrome ou du niobium, ayant la structure MFI, BEA, MOR, TON, MTW, FER, CHA, ERI, RHO, GIS, BOG, NON, EMT, HEU, KFI, FAU, DDR, MTT, RUT, LTL, MAZ, GME, NES, OFF, SGT, EUO, MFS, MCM-22, MEL, une structure mixte MFI/MEL, et les mélanges d'au moins deux d'entre eux.

7. Procédé selon l'une des revendications 1 à 6, dans lequel on utilise en tant que catalyseur zéolitique un catalyseur pouvant être préparé par un procédé comportant les étapes suivantes :
(i) addition, à un mélange contenant une zéolite ou un mélange d'au moins deux d'entre elles, d'un mélange contenant au moins un alcool et de l'eau, et
(ii) malaxage, façonnage, séchage et calcination du mélange additionné selon l'étape (i).

8. Procédé selon l'une des revendications 1 à 7, dans lequel la régénération du catalyseur zéolitique au moins partiellement désactivé selon l'étape (III) est mise en oeuvre comme suit :
(a) chauffage d'un catalyseur au moins partiellement désactivé à une température comprise dans la plage de 250 à 600°C dans une atmosphère contenant moins de 2 % en volume d'oxygène, et
(b) envoi, sur le catalyseur, à une température comprise dans la plage de 250 à 800°C et de préférence de 350 à 600°C, d'un courant gazeux ayant une teneur de 0,1 à 4 % en volume d'une substance cédant de l'oxygène, ou d'oxygène, ou d'un mélange d'au moins deux d'entre elles.

9. Procédé selon l'une des revendications 1 à 8, dans lequel la régénération selon l'étape (III) du catalyseur au moins partiellement désactivé est mise en oeuvre dans un appareillage destiné à la réaction selon l'étape (II), sans que le catalyseur zéolitique soit retiré de cet appareillage.
